# EUROPÄISCHE PATENTSCHRIFT

(11) **EP 2 114 965 B1**
(45) Veröffentlichungstag und Bekanntmachung des Hinweises auf die Patenterteilung: **06.10.2010**
(21) Anmeldenummer: 07856451.5
(22) Anmeldetag: 07.12.2007
(51) Int. Cl.: C07F 9/28, C07F 9/52, H01M 10/08

(54) **VERBINDUNGEN ENTHALTEND ORGANOFLUOROCHLOROPHOSPHATANIONEN**
COMPOUNDS COMPRISING ORGANIC FLUOROCHLORO PHOSPHATE ANIONS
COMPOSÉ CONTENANT DES IONS FLUOROCHLOROPHOSPHATANE ORGANIQUES

(30) Priorität: 31.01.2007 DE 102007004698
(43) Veröffentlichungstag der Anmeldung: 11.11.2009
(73) Patentinhaber: Merck Patent GmbH, 64293 Darmstadt (DE)
(72) Erfinder: IGNATYEV, Nikolai, 47058 Duisburg (DE); BISSKY, German, 59077 Hamm (DE); WILLNER, Helge, 45481 Muehlheim/Ruhr (DE)
(86) Internationale Anmeldenummer: PCT/EP2007/010646
(87) Internationale Veröffentlichungsnummer: WO 2008/092489

(56) Entgegenhaltungen:
- EP-A- 1 162 204
- EP-B1- 0 929 558
- WO-A-02/085919
- WO-A-2006/128563
- US-B1- 6 340 716
- A. N. IVANOV, V. B. SOKOLOV, T. V. GOREVA AND I. V. MARTYNOV: "2,2,3,3-Tetrafluoropropyldichlorophosphit e in the Allen reaction with 1,1-dichloro-1-nitrosoalkanes" JOURNAL OF FLUORINE CHEMISTRY, Bd. 58, Nr. 2-3, 1992, Seite 374, XP002470493

## Beschreibung

Die vorliegende Erfindung betrifft Verbindungen mit Organofluorochlorophosphatanionen, deren Herstellung sowie deren Verwendung, insbesondere als lonische Flüssigkeiten.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf.

Das Gebiet der ionischen Flüssigkeiten wird zur Zeit intensiv erforscht, da die Anwendungsmöglichkeiten vielfältig sind. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "lonic liquids. Green solvent for the future", Pure Appl. Chem., 72 (2000), 1391-1398; P. Wasserscheid, W. Keim "lonische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Die Eigenschaften ionischer Flüssigkeiten, z.B. Schmelzpunkt, thermische und elektrochemische Stabilität, Viskosität, werden stark von der Natur des Anions beeinflusst. Demgegenüber können die Polarität und die Hydrophilie bzw. Lipophilie durch die geeignete Wahl des Kation/Anion-Paares variiert werden. Daher besteht grundsätzlicher Bedarf an neuen ionischen Flüssigkeiten mit variierten Eigenschaften, die zusätzliche Möglichkeiten hinsichtlich ihrer Verwendung ermöglichen.

Aus EP 0 929 558, WO 02/085919 und EP 1 162 204 sind Salze mit Perfluoralkylfluorophosphatanionen (kurz FAP-Anionen) bekannt. Diese Salze zeichnen sich durch eine hohe elektrochemische und thermische Stabilität aus und weisen gleichzeitig eine niedrige Viskosität auf. Salze auf Basis von FAP-Anionen sind weitgehend inert und besitzen eine größere Stabilität gegenüber Hydrolyse als beispielsweise Salze mit PF₆⁻ -Anionen.

Vielfach ist es aber erwünscht, Verbindungen, z.B. als Reaktionsmedium, zur Verfügung zu haben, die nach Durchführung der Reaktion einfach zersetzt werden können, um die Belastung der Umwelt mit biologisch kaum abbaubaren Verbindungen zu verringern.

Es bestand also ein Bedarf an neuen Verbindungen, die beispielsweise als ionische Flüssigkeiten eingesetzt werden können und dabei eine leichtere Abbaubarkeit aufweisen.

Aufgabe der vorliegenden Erfindung ist demgemäß die Bereitstellung neuer Verbindungen, die sich beispielsweise als lonische Flüssigkeiten eignen.

Die vorliegende Aufgabe wird gelöst durch die erfindungsgemäßen Verbindungen, Verfahren zu deren Herstellung sowie deren Verwendung.

Ein erster Gegenstand der vorliegenden Erfindung sind somit Verbindungen enthaltend Organofluorochlorophosphatanionen, vorzugsweise Salze mit Organofluorochlorophosphatanionen. Im Sinne der vorliegenden Erfindung meint "Organo" jeden organischen Rest, beispielsweise aliphatische oder aromatische Organo-Reste, die wiederum selbst substituiert sein können, beispielsweise mit weiteren Organo-Resten oder Resten enthaltend Heteroatome. Bezüglich der Organo-Reste gibt es im Rahmen der vorliegenden Erfindung keine Beschränkung.

Insbesondere handelt es sich bei den Verbindungen gemäß der vorliegenden Erfindung um jene der Formel (I)

[Kt]^{z+} z[(CₙHₘF₂ₙ₋₁₋ₘ)ₓPCl_{y}F_{6-x-y}]⁻ (I)

worin [Kt]^{z+} ein anorganisches oder organisches Kation bedeutet, mit n = 1-12, m = 0 bis 2n+1, x = 1-4, y = 1-4, z = 1-4 und mit der Maßgabe dass x + y ≤ 5 ist. Im Rahmen der vorliegenden Erfindung steht y für die Anzahl der im Anion vorliegenden CI-Anionen. Die Zahl z steht für den Grad der Ladung des Kations und damit für die Anzahl der in den erfindungsgemäßen Verbindungen vorliegenden Anionen. Insgesamt soll die Elektroneutralität der Verbindungen gewährleistet sein.

Vorzugsweise handelt es sich bei den erfindungsgemäßen Verbindungen um jene der Formel (I) mit m = 0 und y = 1. Diese bevorzugten Verbindungen werden durch die allgemeine Formel (Ia) dargestellt:

[Kt]^{z+} z[(CₙF₂ₙ₊₁)ₓPCIF₅₋ₓ]⁻ (Ia)

Insbesondere bevorzugt ist x = 3 und/oder n = 2, 3 oder 4. Ganz besonders bevorzugt sind im Rahmen der vorliegenden Erfindung die Verbindungen ausgewählt aus der Gruppe umfassend [Kt]^{z+} z[(C₂F₅)₃PCIF₂]⁻ [Kt]^{z+} z[(C₃F₇)₃PClF₂]⁻ oder [Kt]^{z+} z[(C₄F₉)₃PClF₂]⁻.

In Bezug auf die Wahl des Kations der Verbindung (I) gemäß der vorliegenden Erfindung gibt es per se keine Einschränkungen. So kann das [Kt]^{z+} ein anorganisches order organisches Kation sein. Vorzugsweise handelt es sich bei den Kationen um organische Kationen und insbesondere bevorzugt sind die organischen Kationen ausgewählt aus der Gruppe umfassend Ammonium-, Phosphonium, Uronium-, Thiouronium-, Guanidiniumkationen oder um heterocyclische Kationen handelt. Beispiele von organischen Kationen sind auch Polyammoniumionen mit einem Grad der Ladung z = 4.

Ammoniumkationen können beispielsweise durch die Formel (1)

[NR₄]⁺ (1),

beschrieben werden, wobei
- R: jeweils unabhängig voneinander
H,
OR', NR'₂, mit der Maßgabe, dass maximal ein Substituent R in Formel (1) OR', NR'₂ ist,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R teilweise oder vollständig mit Halogenen, insbesondere -F und/oder -Cl, oder teilweise mit -OH, -OR', - NR'₂,-CN, -C(O)OH, -C(O)NR'₂, -SO₂NR'₂, -C(O)X, -SO₂OH, -SO₂X, -SR', - S(O)R', -SO₂R',-NO₂, substituiert sein können, und wobei ein oder zwei nicht benachbarte und nicht α-ständige Kohlenstoffatome des R, durch Atome und/oder Atomgruppierungen ausgewählt aus der Gruppe -O-, -S-, - S(O)-, -SO₂-, -SO₂O-, -C(O)-, -C(O)O-, -N⁺R'₂-, -P(O)R'O-, -C(O)NR'-, - SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen sein kann.

Phosphoniumkationen können beispielsweise durch die Formel (2)

[PR²₄]⁺ (2),

beschrieben werden, wobei
- R²: jeweils unabhängig voneinander
H, OR' oder NR'₂
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R² teilweise oder vollständig mit Halogenen, insbesondere -F und/oder -CI, oder teilweise mit -OH, -OR', - NR'₂, -CN, -C(O)OH, -C(O)NR'₂, -SO₂NR'₂, -C(O)X, -SO₂OH, -SO₂X, -SR', -S(O)R', -SO₂R',-NO₂, substituiert sein können, und wobei ein oder zwei nicht benachbarte und nicht α-ständige Kohlenstoffatome des R², durch Atome und/oder Atomgruppierungen ausgewählt aus der Gruppe -O-, -S-, - S(O)-, -SO₂-, -SO₂O-, -C(O)-, -C(O)O-, -N⁺R'₂-, -P(O)R'O-, -C(O)NR'-, - SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

Ausgeschlossen sind jedoch Kationen der Formeln (1) und (2), in denen alle vier oder drei Substituenten R und R² vollständig mit Halogenen substituiert sind, beispielsweise das Tris(trifluormethyl)methylammoniumkation, das Tetra(trifluormethyl)ammoniumkation oder das Tetra(nonafluorbutyl)ammoniumkation.

Uroniumkationen können beispielsweise durch die Formel (3)

[C(R³R⁴N)(OR⁵)(NR⁶R⁷)]⁺ (3),

und Thiouroniumkationen durch die Formel (4),

[(R³R⁴N)(SR⁵)(NR⁶R⁷)]⁺ (4),

beschrieben werden, wobei
- R³ bis R⁷: jeweils unabhängig voneinander
H, wobei H für R⁵ ausgeschlossen wird,
geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -CI, oder teilweise mit -OH, -OR', -NR'₂ , -CN, -C(O)OH, -C(O)NR'₂, -SO₂NR'₂, - C(O)X, -SO₂OH, -SO₂X, -SR', -S(O)R', -SO₂R',-NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, -S(O)-, -SO₂-, -SO₂O-, -C(O)-, - C(O)O-, -N⁺R'₂-, -P(O)R'O-, -C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

Guanidiniumkationen können durch die Formel (5)

[C(NR⁸R⁹)(NR¹⁰R¹¹)(NR¹²R¹³)]⁺ (5),

beschrieben werden, wobei
- R⁸ bis R¹³: jeweils unabhängig voneinander
H, -CN, NR'₂, -OR'
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R⁸ bis R¹³ teilweise
oder vollständig mit Halogenen, insbesondere -F und/oder -CI, oder teilweise mit -OH, -OR', -NR'₂, -CN, -C(O)OH, -C(O)NR'₂, -SO₂NR'₂, - C(O)X, -SO₂OH, -SO₂X, -SR', -S(O)R', -SO₂R',-NO₂, substituiert sein können, und wobei ein oder zwei nicht benachbarte und nicht α-ständige Kohlenstoffatome von R⁸ bis R¹³ durch Atome und/oder Atomgruppierungen ausgewählt aus der Gruppe -O-, -S-, -S(O)-, -SO₂-, - SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

Darüber hinaus können Kationen der allgemeinen Formel (6)

[HetN]^{z+} (6)

eingesetzt werden, wobei
- HetN^{z+}: ein heterocyclisches Kation, ausgewählt aus der Gruppe 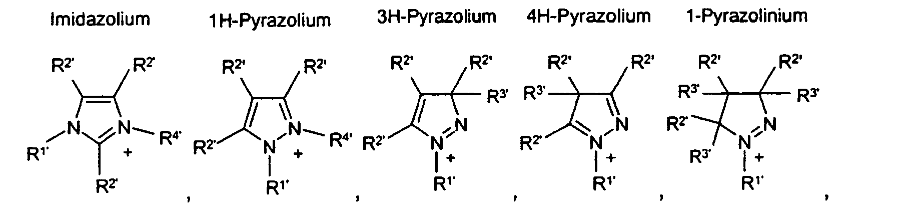 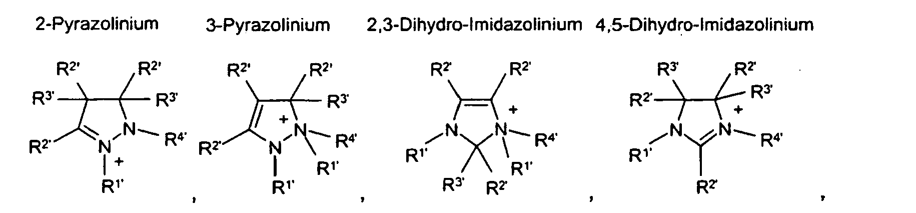 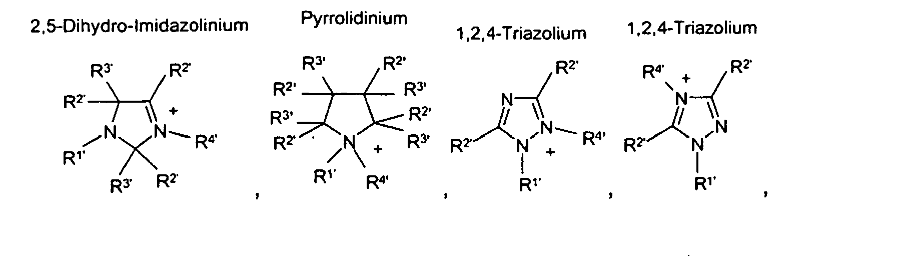 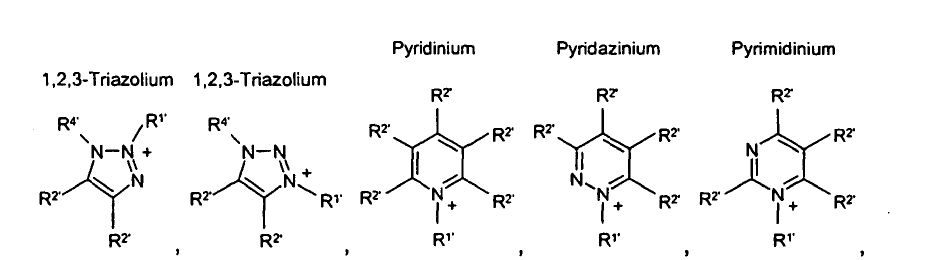 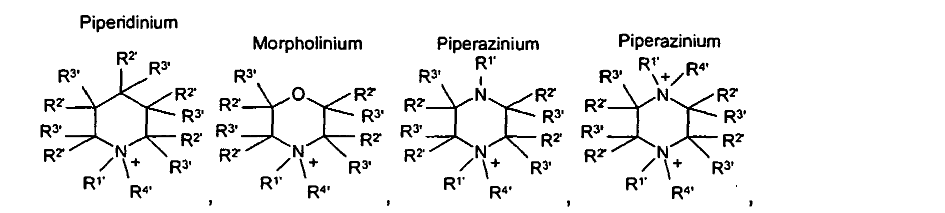 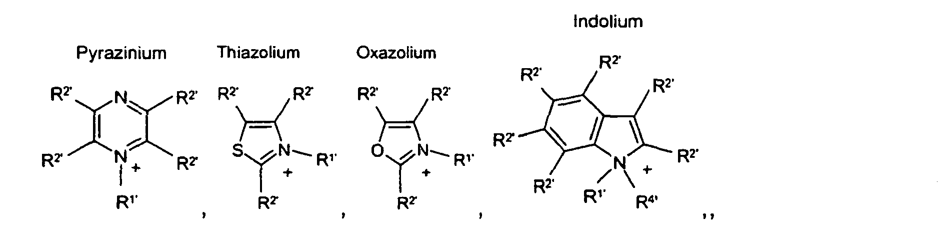 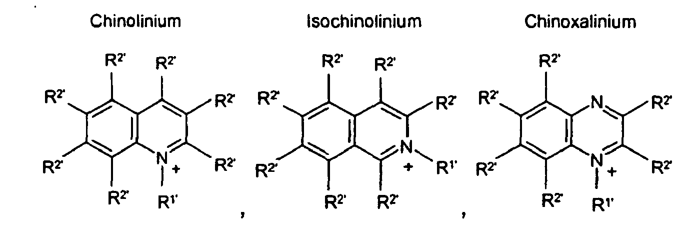 oder 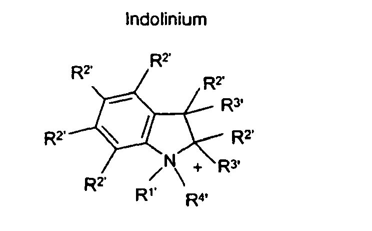
bedeutet, wobei die Substituenten
- R¹, bis R⁴': jeweils unabhängig voneinander
H,
F, CI, Br, J, -CN, -OR', -NR'₂, -P(O)R'₂, -P(O)(OR')₂, -P(O)(NR'₂)₂, -C(O)R', -C(O)OR', -C(O)X, -C(O)NR'₂, -SO₂NR'₂, -SO₂OH, -SO₂X, -SR', -S(O)R', -
SO₂R' und/oder NO₂, mit der Maßgabe, dass R¹', R³', R⁴' H und/oder ein geradkettiges oder verzweigtes Alkyl mit 1-20 C-Atomen, geradkettiges oder verzweigtes Alkenyl mit 2-20 C-Atomen und einer oder mehreren Doppelbindungen ist,
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R¹' bis R⁴' teilweise oder vollständig mit Halogenen, insbesondere -F und/oder -CI, oder -OH, -OR', -NR'₂, -CN, -C(O)OH, -C(O)NR'₂, -SO₂NR'₂, -C(O)X, -SO₂OH, -SO₂X, -SR', -S(O)R', - SO₂R', -NO₂, substituiert sein können, wobei jedoch nicht gleichzeitig R^{1'} und R^{4'} vollständig mit Halogenen substituiert sein dürfen, und wobei ein oder zwei nicht benachbarte und nicht am Heteroatom gebundene Kohlenstoffatome der Substituenten R¹' bis R⁴', durch Atome und/oder Atomgruppierungen ausgewählt aus der -O-, -S-, -S(O)-, -SO₂-, -SO₂O-, - C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

Unter vollständig ungesättigten Substituenten werden im Sinne der vorliegenden Erfindung auch aromatische Substituenten verstanden. Als Substituenten R und R² bis R¹³ der Verbindungen der Formeln (1) bis (5) kommen erfindungsgemäß dabei neben H bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R und R² in den Verbindungen der Formel (1) oder (2) können dabei gleich oder verschieden sein. Bevorzugt sind die Substituenten R und R² verschieden.

Die Substituenten R und R² sind insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations
[C(NR⁸R⁹)(NR¹⁰R¹¹)(NR¹²R¹³)]⁺ können auch paarweise derart verbunden sein, dass mono-, bi- oder polycyclische Kationen entstehen.

Ohne Einschränkung der Allgemeinheit sind Beispiele für solche Guanidinium-Kationen: 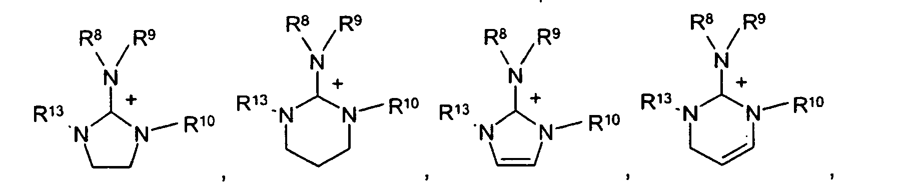 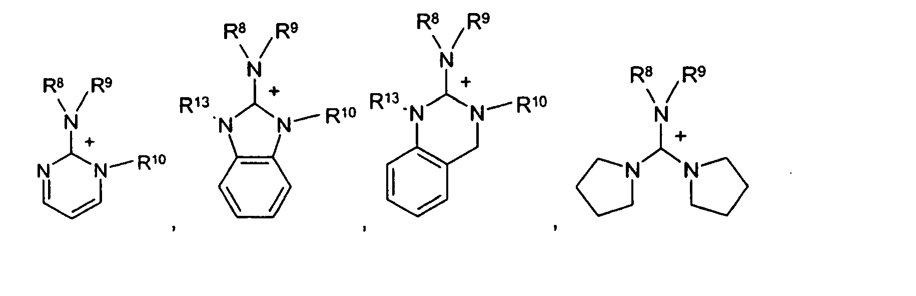 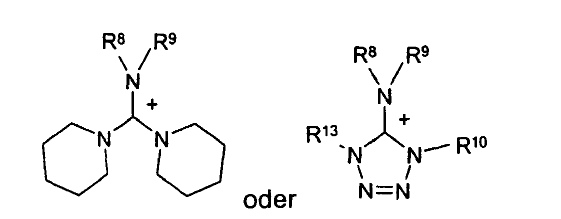 wobei die Substituenten R⁸ bis R¹⁰ und R¹³ eine zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, -CN, -NO₂, F, Cl, Br, I, -OH, -C₁-C₆-Alkoxy, -NR'₂ ,-SR', -S(O)R', -SO₂R', -COOH, -SO₂NR'₂, -SO₂X' oder -SO₃H substituiert sein, wobei X und R' eine zuvor angegebene Bedeutung haben, substituiertes oder unsubstituiertes Phenyl oder unsubstituierter oder substituierter Heterocyclus substituiert sein.

Bis zu vier Substituenten des Uroniumkations [C(R³R⁴N)(OR⁵)(NR⁶R⁷)]⁺ oder des Thiouroniumkations [C(R³R⁴N)(SR⁵)(NR⁶R⁷)]⁺ können auch paarweise derart verbunden sein, dass mono-, bi- oder polycyclische Kationen entstehen.

Ohne Einschränkung der Allgemeinheit sind Beispiele für solche Kationen im Folgenden angegeben, wobei Y = O oder S bedeutet: 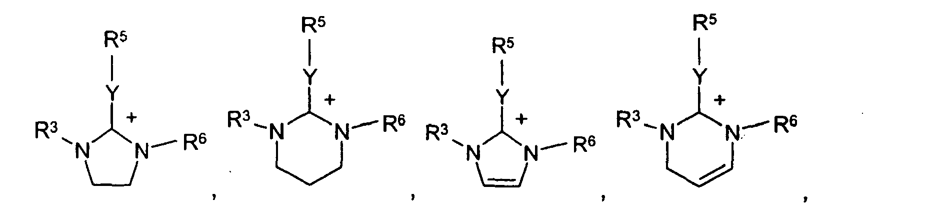 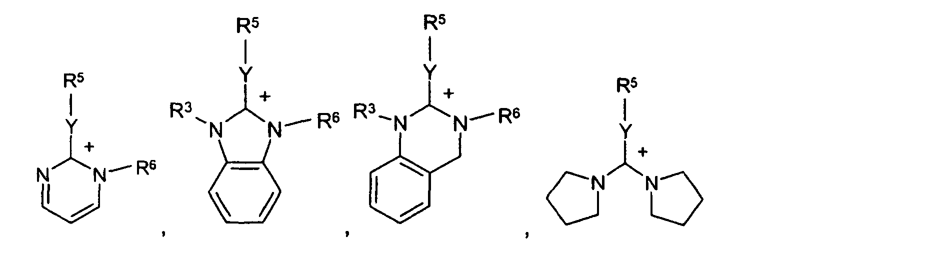 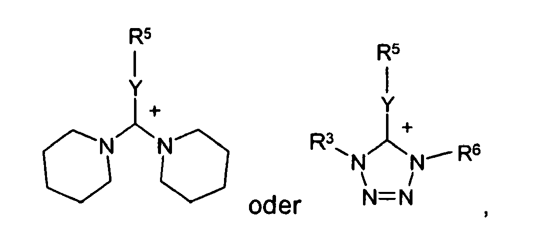 wobei die Substituenten R³, R⁵ und R⁶ eine zuvor angegebene oder besonders bevorzugte Bedeutung haben können. Gegebenenfalls können die Carbocyclen oder Heterocyclen der zuvor angegebenen Kationen noch durch C₁- bis C₆-Alkyl, C₁- bis C₆-Alkenyl, -CN, -NO₂, F, Cl, Br, I, -OH, -C₁-C₆-Alkoxy, -NR'₂ , -SR', -S(O)R', -SO₂R', -COOH, SO₂NR'₂, SO₂X oder SO₃H oder substituiertes oder unsubstituiertes Phenyl oder unsubstituierter oder substituierter Heterocyclus substituiert sein, wobei X und R' eine zuvor angegebene Bedeutung haben.

Die Substituenten R³ bis R¹³ sind jeweils unabhängig voneinander bevorzugt eine geradkettige oder verzweigte Alkylgruppe mit 1 bis 16 C-Atomen. Die Substituenten R³ und R⁴, R⁶ und R⁷, R⁸ und R⁹, R¹⁰ und R¹¹ und R¹² und R¹³ in Verbindungen der Formeln (3) bis (5) können dabei gleich oder verschieden sein. Besonders bevorzugt sind R³ bis R¹³ jeweils unabhängig voneinander Methyl, Ethyl, n-Propyl, Isopropyl, n-Butyl, tert.-Butyl, sek.-Butyl, Phenyl, Hexyl oder Cyclohexyl, ganz besonders bevorzugt Methyl, Ethyl, n-Propyl, Isopropyl, n-Butyl oder Hexyl.

Als Substituenten R^{1'} bis R^{4'} von Verbindungen der Formel (6) kommen erfindungsgemäß dabei neben H bevorzugt in Frage: C₁- bis C₂₀, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R^{1'} und R^{4'} sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Cyclohexyl, Phenyl oder Benzyl. Sie sind ganz besonders bevorzugt Methyl, Ethyl, n-Butyl oder Hexyl. In Pyrrolidinium-, Piperidinium- oder Indolinium-Verbindungen sind die beiden Substituenten R^{1'} und R^{4'} bevorzugt unterschiedlich.

Der Substituent R^{2'} oder R^{3'} ist jeweils unabhängig voneinander insbesondere H, Methyl, Ethyl, Isopropyl, Propyl, Butyl, sek.-Butyl, tert.-Butyl, Cyclohexyl, Phenyl oder Benzyl. Besonders bevorzugt ist R^{2'} H, Methyl, Ethyl, Isopropyl, Propyl, Butyl oder sek.-Butyl. Ganz besonders bevorzugt sind R^{2'} und R^{3'} H.

Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl. Gegebenenfalls Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -OH, -OR', -NR'₂, -CN, -C(O)OH, -C(O)NR'₂, -SO₂NR'₂, -C(O)X, -SO₂OH, -SO₂X, -SR', -S(O)R', -SO₂R',-NO₂ substituiert sein können.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder mit -OH, -OR', -NR'₂, -CN, - C(O)OH, -C(O)NR'₂, -SO₂NR'₂, -C(O)X, -SO₂OH, -SO₂X, -SR', -S(O)R', -SO₂R', -NO₂ substituiert sein kann.

In den Substituenten R, R² bis R¹³ oder R^{1'} bis R^{4'} können auch ein oder zwei nicht benachbarte und nicht α-ständig zum Heteroatom gebundene Kohlenstoffatome, durch Atome und/oder Atomgruppierungen ausgewählt aus der Gruppe -O-, -S-, -S(O)-, -SO₂-, -SO₂O-, -C(O)-, -C(O)O-, -N⁺R'₂-, - P(O)R'O-, -C(O)NR'-, -SO₂NR'-, -OP(O)R'O-, -P(O)(NR'₂)NR'-, -PR'₂=N-oder -P(O)R'- ersetzt werden, mit R' = nicht, teilweise oder perfluoriertes C₁- bis C₁₈-Alkyl, C₃- bis C₇-Cycloalkyl, unsubstituiertes oder substituiertes Phenyl.

Ohne Einschränkung der Allgemeinheit sind Beispiele für derart modifizierte Substituenten R, R² bis R¹³ und R^{1'} bis R^{4'}:
-OCH₃, -OCH(CH₃)₂, -CH₂OCH₃, -CH₂-CH₂-O-CH₃, -C₂H₄OCH(CH₃)₂, - C₂H₄SC₂H₅, -C₂H₄SCH(CH₃)₂, -S(O)CH₃, -SO₂CH₃, -SO₂C₆H₅, -SO₂C₃H₇, - SO₂CH(CH₃) ₂, -SO₂CH₂CF₃, -CH₂SO₂CH₃, -O-C₄H₈-O-C₄H₉, -CF₃, -C₂F₅, C₃F₇, -C₄F₉, -C(CF₃)₃, -CF₂SO₂CF₃, -C₂F₄N(C₂F₅)C₂F₅, -CHF₂, -CH₂CF₃, - C₂F₂H₃, -C₃FH₆, -CH₂C₃F₇, -C(CFH₂)₃, -CH₂C(O)OH, -CH₂C₆H₅, -C(O)C₆H₅ oder P(O)(C₂H₅)₂.
In R' ist C₃- bis C₇-Cycloalkyl beispielsweise Cyclopropyl, Cyclobutyl, Cyclopentyl, Cyclohexyl oder Cycloheptyl.
In R' bedeutet substituiertes Phenyl, durch C₁- bis C₆-Alkyl, C₁- bis C₆-Alkenyl, -CN, -NO₂, F, Cl, Br, I, -OH, -C₁-C₆-Alkoxy, NR"₂, -COOH, -SO₂X', -SR", -S(O)R", -SO₂R", SO₂NR"₂ oder SO₃H substituiertes Phenyl, wobei X' F, CI oder Br und R" ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m- oder p-Isopropylphenyl, o-, m- oder p-tert.-Butylphenyl, o-, m- oder p-Nitrophenyl, o-, m- oder p-Hydroxyphenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-lodphenyl, weiter bevorzugt 2,3-, 2,4-, 2,5-, 2,6-, 3,4- oder 3,5-Dimethylphenyl, 2,3-, 2,4-, 2,5- , 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.
In R^{1'} bis R^{4'} wird als Heteroaryl ein gesättigter oder ungesättigter mono- oder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁- bis C₆-Alkenyl, -CN, -NO₂, F, Cl, Br, I, -OH, -NR"₂, -C₁-C₆-Alkoxy, -COOH, -SO₂X', -SO₂NR"₂, -SR", -S(O)R", -SO₂R" oder SO₃H substituiert sein kann, wobei X' und R" eine zuvor angegebene Bedeutung haben.

Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinotinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Unter Heteroaryl-C₁-C₆-alkyl wird nun in Analogie zu Aryl-C₁-C₆-alkyl beispielsweise Pyridinyl-methyl, Pyridinyl-ethyl, Pyridinyl-propyl, Pyridinylbutyl, Pyridinyl-pentyl, Pyridinyl-hexyl verstanden, wobei weiterhin die zuvor beschriebenen Heterocyclen in dieser Weise mit der Alkylenkette verknüpft werden können.

HetN^{z+} ist bevorzugt 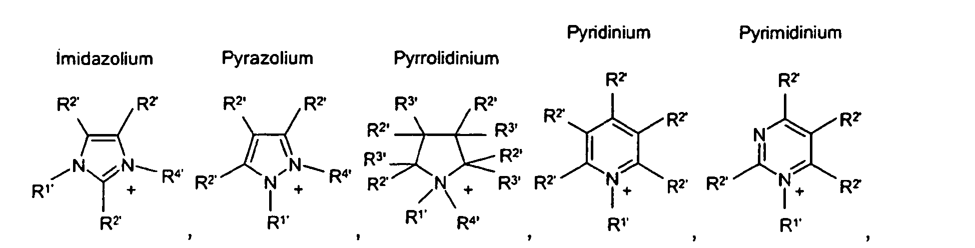 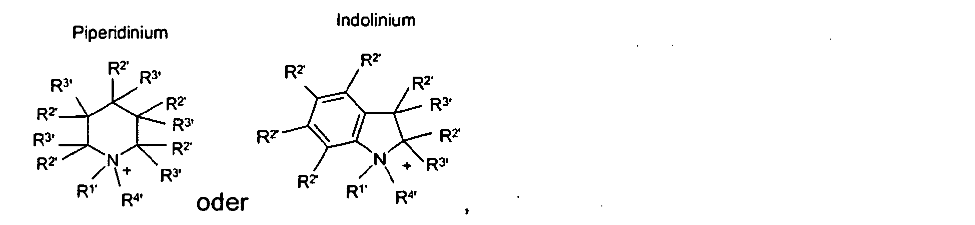 wobei die Substituenten R^{1'} bis R^{4'} jeweils unabhängig voneinander eine zuvor beschriebene Bedeutung haben.

In einer weiteren Ausführungsform der vorliegenden Erfindung kann das Kation [Kt]^{z+}ein [(R°)₃O]⁺-Kation oder ein [(R°)₃S]⁺-Kation sein, wobei R° geradkettige oder verzweigte Alkylgruppen mit 1-8 C-Atomen oder unsubstituiertes Phenyl oder mit R°, OR°, N(R°)₂, CN oder Halogen substituiertes Phenyl bedeutet.

R° des [(R°)₃O]⁺-Kations oder [(R°)₃S]⁺-Kations ist vorzugsweise geradkettiges Alkyl mit 1-8 C-Atomen, bevorzugt mit 1-4 C-Atomen, insbesondere Methyl oder Ethyl, ganz besonders bevorzugt Ethyl.

Das Kation [Kt]^{z+} kann darüber hinaus auch anorganisch sein, insbesondere handelt es sich dabei um ein Metallkation. Das Metallkation kann Metalle der Gruppen 1 bis 12 des Periodensystems umfassen, insbesondere Alkalimetalle. Vorzugsweise ist das Metall ausgewählt aus der Gruppe umfassend Li, K, Rb, Cs.

Vorzugsweise handelt es sich bei den Kationen der erfindungsgemäßen Verbindungen um Ammonium-, Phosphonium-, Guanidinium- oder heterocyclische Kationen, insbesondere bevorzugt um heterocyclische Kationen (HetN^{z+}). HetN^{z+} ist besonders bevorzugt Imidazolium, Pyrrolidinium oder Pyridinium, wie zuvor definiert, wobei die Substituenten R^{1'} bis R^{4'} jeweils unabhängig voneinander eine zuvor beschriebene Bedeutung haben. HetN^{z+} ist ganz besonders bevorzugt Imidazolium, wobei die Substituenten R^{1'} bis R^{4'} jeweils unabhängig voneinander eine zuvor beschriebene Bedeutung haben. Ganz besonders bevorzugt handelt es sich bei den Verbindungen gemäß der Formel (I) um Imidazolium-, Pyrrolidinium-, Pyridinium-, Ammonium-, Phosphonium-, Guanidinium-Salze mit [(C₂F₅)₃PCIF₂]⁻-, [(C₃F₇)₃PCIF₂]⁻- oder [(C₄F₉)₃PCIF₂]⁻-Anionen.

Es versteht sich für den Fachmann von selbst, dass in den erfindungsgemäßen Verbindungen Substituenten wie beispielsweise C, H, N, O, CI, F durch die entsprechenden Isotope ersetzt sein können.

Ebenfalls Gegenstand der vorliegenden Erfindung sind Verfahren zur Herstellung von Verbindungen mit Organofluorochlorophosphatanionen umfassend die Umsetzung eines Chloridsalzes mit Organofluorophosporanen. Vorzugsweise handelt es sich bei den erfindungsgemäßen Verfahren um jene zur Herstellung einer Verbindung der Formel (I), wie oben beschrieben, umfassend die Umsetzung von [Kt]^{z+}z[Cl]⁻ mit einer Verbindung der allgemeinen Formel (II)

(CₙHₘF₂ₙ₊₁₋ₘ)×PF₅₋ₓ (II)

worin [Kt]^{z+} ein anorganisches oder organisches Kation mit z = 1-4 und in Verbindungen der Formel II n = 1-12, m = 0 bis 2n+1, x = 1-4 und y = 1-4 bedeutet. Vorzugsweise ist y = 1 und m = 0, das heißt bevorzugt wird [Kt]⁺CI⁻ mit einer Verbindung der allgemeinen Formel (IIa) umgesetzt

(CₙF₂ₙ₊₁)ₓPF₅₋ₓ (IIa)

worin [Kt]⁺ ein anorganisches oder organisches Kation bedeutet und in Formel IIa n = 1-12 und x = 1-4 bedeutet.

Die Herstellung von Perfluoralkylphosphoranen (Formel (II) bzw. (IIa)) als Ausgangsverbindungen für das erfindungsgemäße Verfahren ist dem Fachmann aus dem Stand der Technik geläufig, beispielsweise aus der deutschen Patentanmeldung DE 19 846 636 A1, die hiermit als Referenz eingeführt wird und somit als Teil der Offenbarung gilt.

Das erfindungsgemäße Verfahren kann bei Temperaturen von -40 bis 180°C durchgeführt werden, vorzugsweise erfolgt die Umsetzung bei 0 bis 50°C und ganz besonders bevorzugt bei Raumtemperatur.

Die Umsetzung erfolgt dabei insbesondere in einem Lösungsmittel, kann aber auch in Abwesenheit eines Lösungsmittels durchgeführt werden.

Geeignete Lösungsmittel sind jene ausgewählt aus der Gruppe der Nitrile, Dialkylcarbonate, Glymes, Dialkylethers, Cyclic Ethers, Dimethylfomamide, Dimethylsulfoxide, Dichlormethane, Wasser oder Mischungen hieraus. Bevorzugte Beispiele geeigneter Lösungsmittel sind Acetonitril und Glymes.

Die Reaktionsdauer beträgt üblicherweise 5 Minuten bis 24 Stunden, vorzugsweise 1 Stunde bis 10 Stunden.

Nach erfolgter Umsetzung können die erfindungsgemäßen Verbindungen in für den Fachmann bekannter Weise aufgearbeitet und aufgereinigt werden, beispielsweise durch Entfernen flüchtiger Bestandteile im Vakuum und Trocknen, wobei gegebenenfalls weitere Reinigungsschritte nachfolgen können.

Viele der genannten erfindungsgemäßen Verbindungen eignen sich zur Verwendung als Ionische Flüssigkeit bzw. sind vorzugsweise lonische Flüssigkeiten und können somit in einer ganzen Reihe von Anwendungssystemen angewendet werden.

Weiterhin ist Gegenstand der vorliegenden Erfindung die Verwendung der erfindungsgemäßen Verbindungen als Lösungsmittel oder Lösungsmittelzusatz, als Phasentransferkatalysator, als Extraktionsmittel, als Wärmeträger, als oberflächenaktive Substanz, als Weichmacher, als Flammschutzmittel, als Additiv oder als Leitsalz und Katalysatoren in chemischen Prozessen.

Im Falle der Verwendung der genannten Verbindungen als Lösungsmittel eignen sich diese in jeder dem Fachmann bekannten Art von Reaktion, z.B. für Übergangsmetall- oder Enzym-katalysierte Reaktionen, wie beispielsweise Hydroformylierungsreaktionen, Friedel-Crafts-Reaktionen, Oligomerisierungsreaktionen, Veresterungen oder Isomerisierungsreaktionen, wobei die genannte Liste nicht abschließend ist.

Bei Verwendung als Extraktionsmittel können die Verbindungen zur Abtrennung von Reaktionsprodukten aber auch zur Abtrennung von Verunreinigungen eingesetzt werden, je nachdem wie die Löslichkeit der jeweiligen Komponente in der verwendeten erfindungsgemäßen Verbindung ist. Darüber hinaus können die Verbindungen der Formel (I) auch als Trennmittel bei der Separation mehrerer Komponenten dienen, beispielsweise bei der destillativen Trennung mehrerer Komponenten eines Gemisches.

Weitere Anwendungsmöglichkeiten sind die Anwendung als Weichmacher in Polymermaterialien, als Flammschutzmittel für eine Reihe von Materialien oder Anwendungen sowie als Leitsalz in unterschiedlichen elektrochemischen Zellen und Anwendungen, z.B. in galvanischen Zellen, in Kondensatoren oder in Brennstoffzellen.

Elektrolyte, elektrochemische Zellen, Kondensatoren oder Brennstoffzellen enthaltend mindestens eine erfindungsgemäße Verbindung der allgemeinen Formel (I) sind ebenfalls Gegenstand der vorliegenden Erfindung. In den genannten Anwendungen können die erfindungsgemäßen Verbindungen in Kombination mit allen dem Fachmann bekannten Materialien und Zusätzen angewendet werden, ohne dass es einem erfinderischen Zutun des Fachmanns bedarf.

Darüber hinaus eignen sich die erfindungsgemäßen Verbindungen zur Herstellung von Organophosphinaten. Im Fall von Wasser als Lösungsmittel können die zuerst entstehenden Organofluorochlorophosphate weiter mit Wasser reagieren, unter Bildung korrespondierender Organophosphinate. Alternativ können die Organophosphinate auch hergestellt werden, in dem die erfindungsgemäß hergestellten Organofluorochlorophosphate in Wasser gelöst werden. Die erfindungsgemäßen Organofluorochlorophosphate eignen sich dementsprechend in der Verwendung zur Herstellung von Organophosphinaten. Diese Organophosphinate stellen selbst wieder wertvolle Verbindungen dar, die sich beispielsweise als lonische Flüssigkeiten einsetzen lassen. Entsprechende Organophosphinate sind beispielsweise aus WO 2003/087110 bekannt, deren Offenbarung hiermit durch Referenz eingefügt ist.
Erfindungsgemäße Verfahren zur Herstellung von Organophosphinaten umfassen die Umsetzung einer Verbindung gemäß der vorliegenden Erfindung mit Wasser oder Wasser enthaltenden Lösungsmitteln oder Lösungsmittelgemischen. Diese Umsetzung kann bei Temperaturen von-40 bis 200°C, bei atmosphärischem Druck bis hin zu Drücken von 100 bar, erfolgen. Vorzugsweise erfolgt die Umsetzung mit Wasser bei Raumtemperatur und atmosphärischen Druck.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Die ¹H-, ¹⁹F- und ³¹P-NMR-Spektren werden an einem Bruker ARX 400 Spektrometer (400.13 MHz für ¹H, 376.46 für ¹⁹F und 161.98 für ³¹P) in Acetonitril-D₃ gemessen, falls nicht anders in den Beispielen angegeben. CCl₃F und TMS werden als interne Referenz bei der Vermessung der ¹⁹F NMR- und Protonen-NMR-Spektren eingesetzt. Für die ³¹P NMR-Spektren wird als externe Referenz 85%-ige H₃PO₄ in D₂O in Acetonitril-D₃ bei einer Frequenz von 230.11 Hz in einem separaten Experiment vermessen.

### Beispiele

### Beispiel 1: Tetrabutylammonium-tris(pentafluoroethyl)difluorochlorophosphat

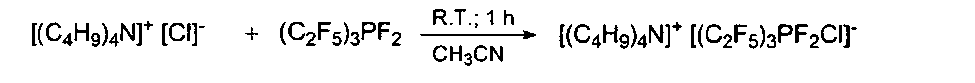

Eine Mischung aus 7.04 g (25.3 mmol) Tetrabutylammoniumchlorid und 12.0 g (28.2 mmol) Tris(pentafluoroethyl)difluorophosphoran werden in 20 ml Acetonitril gelöst und bei Raumtemperatur für eine Stunde gerührt. Flüchtige Bestandteile (Überschüsse an Tris(pentafluoroethyl)difluorophosphoran und Acetonitril) werden im Vakuum entfernt und der Rückstand wird im Vakuum für zwei Stunden bei 7 Pa und bei 40-50°C (Temperatur des Ölbades) getrocknet. 17.0 g eines viskosen, öligen Materials werden erhalten. Die Ausbeute an Tetrabutylammoniumtris(pentafluoroethyl)difluoro-chlorophosphate beträgt 95.5 %, bezogen auf das eingesetzte Tetrabutylammoniumchlorid. Die Verbindung wird NMR-spektroskopisch analysiert.

NMR-Daten:
¹H NMR-Spektrum, ppm: 0.96 t (CH₃), 1.35 t,q (CH₂), 1.59 m (CH₂), 3.06 m (CH₂); J³_{H,H} = 7.4 Hz.
¹⁹F NMR-Spektrum, ppm: -25.52 d,m (PF), -69.83 d,m (PF), -77.49 t (CF₃), -80.04 m (2CF₃), -105.12 d,d (CF₂, F_{A}), -105.87 d,d (CF₂, F_{B}), -108.89 d,m (CF₂), -114.72 d,m (CF₂, F_{A}), -115.45 d,m (CF₂, F_{B}), J¹_{P,F} = 933 Hz, J¹_{P,F} = 863 Hz, J²_{P,F} = 98 Hz, J²_{P,F} = 82 Hz, J²_{P,F} = 116 Hz, J²_{A,B} = 281 Hz, J⁴_{F,F} = 21 Hz,.
³¹P NMR-Spektrum, ppm: -147.4 d,d,m.

### Beispiel 2: Benzyltriethylammonium-tris(pentafluoroethyl)difluorochlorophosphat

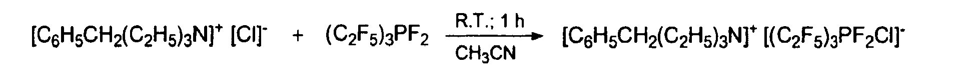

Eine Mischung aus 11.45 g (50.3 mmol) Benzyltriethylammoniumchlorid und 23.6 g (55.4 mmol) Tris(pentafluoroethyl)difluorophosphoran werden in 30 ml Acetonitril gelöst und bei Raumtemperatur für eine Stunde gerührt. Flüchtige Bestandteile (Überschüsse an Tris(pentafluoroethyl)difluorophosphoran und Acetonitril) werden im Vakuum entfernt und der Rückstand wird im Vakuum für zwei Stunden bei 7 Pa und bei 40-50°C (Temperatur des Ölbades) getrocknet. 32.3 g eines viskosen, öligen Materials werden erhalten. Die Ausbeute an Benzyltriethylammonium-tris(pentafluoroethyl)difluorochlorophosphat beträgt 98.2 %, bezogen auf das eingesetzte Benzyltriethylammoniumchlorid. Die Verbindung wird NMR-spektroskopisch analysiert.

NMR-Daten:
¹H NMR-Spektrum, ppm: 1.34 t,t (3CH₃), 3.14 q (3CH₂), 4.30 s (CH₂), 7.44-7.58 m (C₆H₅); J³_{H,H} = 7.3 Hz.
¹⁹F NMR-Spektrum, ppm: -25.52 d,m (PF), -69.81 d,m (PF), -77.47 t (CF₃), -80.03 m (2CF₃), -105.11 d,d (CF₂, F_{A}), -105.85 d,d (CF₂, F_{B}), -108.87 d,m (CF₂), -114.72 d,m (CF₂, F_{A}), -115.45 d,m (CF₂, F_{B}), J¹_{P,F} = 928 Hz, J¹_{P,F} = 861 Hz, J²_{P,F} = 99 Hz, J²_{P,F} = 82 Hz, J²_{P,F} = 116 Hz, J²_{A,B} = 280 Hz, J⁴_{F,F} = 21 Hz,.
³¹P NMR-Spektrum, ppm: -147.4 d,d,m.

### Beispiel 3: 1-Ethyl-3-methylimidazolium-tris(pentafluoroethyl)difluorochlorophosphat

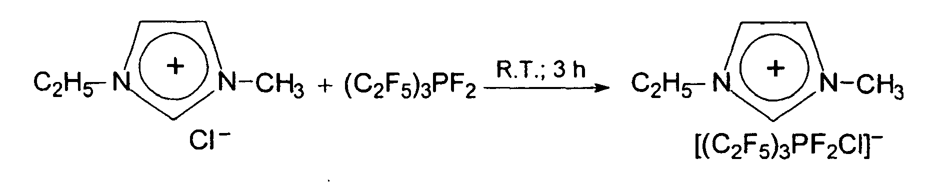

Eine Mischung aus 4.1 g (28.0 mmol) 1-Ethyl-3-methylimidazoliumchlorid und 13.1 g (30.8 mmol) Tris(pentafluoroethyl)difluorophosphoran wird bei Raumtemperatur für drei Stunden gerührt. Flüchtige Bestandteile (Überschüsse an Tris(pentafluoroethyl)difluorophosphoran) werden im Vakuum entfernt und der Rückstand wird im Vakuum für zwei Stunden bei 7 Pa und bei 40-50°C (Temperatur des Ölbades) getrocknet. 15.9 g eines viskosen, öligen Materials werden erhalten. Die Ausbeute an 1-Ethyl-3-methylimidazolium-tris(pentafluoroethyl)difluorochlorophosphat beträgt 99.2 %, bezogen auf das eingesetzte 1-Ethyl-3-methylimidazoliumchlorid. Die Verbindung wird NMR-spektroskopisch analysiert.

NMR-Daten:
¹H NMR-Spektrum, ppm: 1.45 t (CH₃); 3.83 s (CH₃); 4.17 q (CH₂); 7.37 m (CH); 7.43 m (CH); 8.57 br. s. (CH); ³J_{H,H} = 7.3 Hz.
¹⁹F NMR-Spektrum, ppm: -25.52 d,m (PF), -69.83 d,m (PF), -77.48 t (CF₃), -80.04 m (2CF₃), -105.12 d,d (CF₂, F_{A}), -105.87 d,d (CF₂, F_{B}), -108.87 d,m (CF₂), -114.72 d,m (CF₂, F_{A}), -115.47 d,m (CF₂, F_{B}), J¹_{P,F} = 933 Hz, J¹_{P,F} = 863 Hz, J²_{P,F} = 98 Hz, J²_{P,F} = 84 Hz, J²_{P,F} = 119 Hz, J²_{A,B} = 281 Hz, J⁴_{F,F} = 21 Hz,.
³¹P NMR-Spektrum, ppm: -147.4 d,d,m.

### Beispiel 4: 1-Butyl-1-methylpyrrolidinium-tris(pentafluoroethyl)difluorochlorophosphat

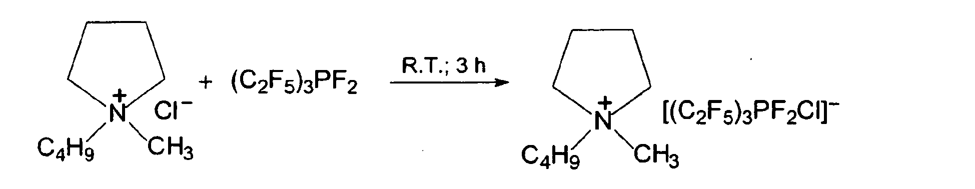

Eine Mischung aus 4.03 g (22.7 mmol) 1-Butyl-1-methylpyrrolidiniumchlorid und 10.8 g (25.4 mmol) Tris(pentafluoroethyl)difluorophosphoran wird bei Raumtemperatur für drei Stunden gerührt. Flüchtige Bestandteile (Überschüsse an Tris(pentafluoroethyl)difluorophosphoran) werden im Vakuum entfernt und der Rückstand wird im Vakuum für zwei Stunden bei 7 Pa und bei 40-50°C (Temperatur des Ölbades) getrocknet. 13.6 g eines viskosen, öligen Materials werden erhalten. Die Ausbeute an 1-Butyl-1-methylpyrrolidinium-tris(pentafluoroethyl)difluorochlorophosphat beträgt 99.2 %, bezogen auf das eingesetzte 1-Butyl-1-methylpyrrolidiniumchlorid. Die Verbindung wird NMR-spektroskopisch analysiert.

NMR-Daten:
¹H NMR-Spektrum, ppm: 0.96 t (CH₃), 1.36 t,q (CH₂), 1.69 m (CH₂), 2.14 m (2CH₂), 2.93 s (CH₃), 3.21 m (CH₂), 3.39 m (2CH₂); J³_{H,H} = 7.4 Hz.
¹⁹F NMR-Spektrum, ppm: -25.52 d,m (PF), -69.81 d,m (PF), -77.48 t (CF₃), -80.03 m (2CF₃), -105.13 d,d (CF₂, F_{A}), -105.87 d,d (CF₂, F_{B}), -108.89 d,m (CF₂), -114.72 d,m (CF₂, F_{A}), -115.45 d,m (CF₂, F_{B}), J¹_{P,F} = 931 Hz, J¹_{P,F} = 861 Hz, J²_{P,F} = 98 Hz, J²_{P,F} = 82 Hz, J²_{P,F} = 116 Hz, J²_{A,B} = 281 Hz, J⁴_{F,F} = 21 Hz,.
³¹P NMR-Spektrum, ppm: -147.4 d,d,m.

### Beispiel 5: Trihexyltetradecylphosphonium-tris(pentafluoroethyl)difluorochlorophosphat

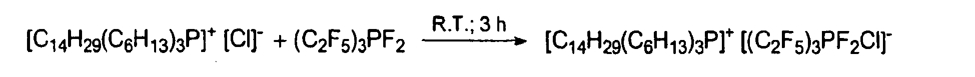

Eine Mischung aus 6.57 g (12.7 mmol) Trihexyltetradecylphosphoniumchlorid und 5.92 g (13.9 mmol) Tris(pentafluoroethyl)difluorophosphoran wird bei Raumtemperatur für drei Stunden gerührt. Flüchtige Bestandteile (Überschüsse an Tris(pentafluoroethyl)difluorophosphoran) werden im Vakuum entfernt und der Rückstand wird im Vakuum für zwei Stunden bei 7 Pa und bei 40-50°C (Temperatur des Ölbades) getrocknet. 11.96 g eines viskosen, öligen Materials werden erhalten. Die Ausbeute an Trihexyltetradecylphosphonium-tris(pentafluoroethyl)difluorochlorophosphat beträgt 99.6 %, bezogen auf das eingesetzte Trihexyltetradecylphosphoniumchlorid. Die Verbindung wird NMR-spektroskopisch analysiert.

NMR-Daten:
¹H NMR-Spektrum, ppm: 0.90 m (4CH₃), 1.28 m (8CH₂), 1.31 m (8CH₂), 1.37-1.55 m (8CH₂), 1.97-2.07 m (4CH₂).
¹⁹F NMR-Spektrum, ppm: -25.53 d,m (PF), -69.85 d,m (PF), -77.50 t (CF₃), -80.06 m (2CF₃), -105.15 d,d (CF₂, F_{A}), -105.89 d,d (CF₂, F_{B}), -108.91 d,m (CF₂), -114.76 d,m (CF₂, F_{A}), -115.49 d,m (CF₂, F_{B}), J¹_{P,F} = 931 Hz, J¹_{P,F} = 861 Hz, J²_{P,F} = 98 Hz, J²_{P,F} = 84 Hz, J²_{P,F} = 123 Hz, J²_{A,B} = 280 Hz, J⁴_{F,F} = 21 Hz,.
³¹P NMR-Spektrum, ppm: 33.4 (1P), -147.4 d,d,m (1P).

### Beispiel 6: Tetrakis(dimethylamino)ethylidenium-di[tris(pentafluoroethyl)-difluorochlorophosphat]

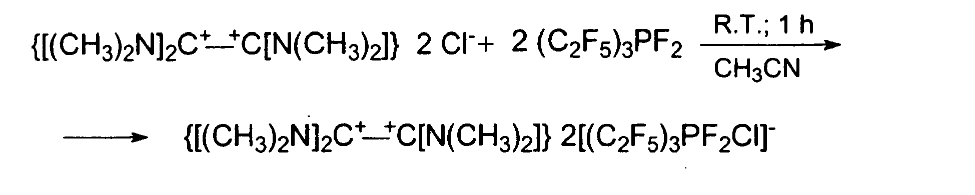

Eine Mischung aus 1.6 g (5.9 mmol) Tetrakis(dimethylamino)ethylideniumdichlorid und 5.6 g (13.1 mmol) Tris(pentafluoroethyl)difluorophosphoran werden in 20 ml Acetonitril gelöst und bei Raumtemperatur für eine Stunde gerührt. Flüchtige Bestandteile (Überschüsse an Tris(pentafluoroethyl)difluorophosphoran und Acetonitril) werden im Vakuum entfernt und der Rückstand wird im Vakuum für zwei Stunden bei 7 Pa und bei 40-50°C (Temperatur des Ölbades) getrocknet. 6.6g eines viskosen, öligen Materials werden erhalten. Die Ausbeute an Tetrakis(dimethylamino)ethylidenium-di[tris(pentafluoroethyl)difluorochlorophosphat] beträgt 99.7 %, bezogen auf das eingesetzte Tetrakis(dimethylamino)ethylideniumdichlorid. Die Verbindung wird NMR-spektroskopisch analysiert.

NMR-Daten:
¹H NMR-Spektrum, ppm: 3.14 s (2CH₃), 3.43 s (2CH₃).
¹⁹F NMR-Spektrum, ppm: -25.52 d,m (PF), -69.83 d,m (PF), -77.46 t (CF₃), -80.00 m (2CF₃), -105.07 d,d (CF₂, F_{A}), -105.82 d,d (CF₂, F_{B}), -108.85 d,m (CF₂), -114.68 d,m (CF₂. F_{A}), -115.45 d,m (CF₂, F_{B}), J¹_{P,F} = 933 Hz, J¹_{P,F} = 861 Hz, J²_{P,F} = 96 Hz, J²_{P,F} = 82 Hz, J²_{P,F} = 120 Hz, J²_{A,B} = 282 Hz, J⁴_{F,F} = 21 Hz,.
³¹P NMR-Spektrum, ppm: -147.3 d,d,m.

### Beispiel 7: 1-Butyl-1-methylpyrrolidinium-tris(heptafluoropropyl)difluorochlorophosphat

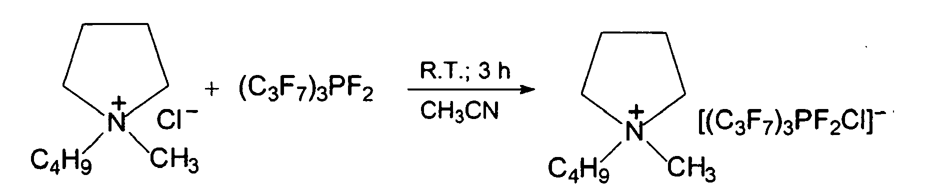

Eine Mischung aus 1.10 g (6.19 mmol) 1-Butyl-1-methylpyrrolidiniumchlorid und 3.57 g (6.19 mmol) Tris(heptafluoropropyl)difluorophosphoran werden in 5 ml Acetonitril gelöst und bei Raumtemperatur für drei Stunden gerührt. Acetonitril werden im Vakuum entfernt und der Rückstand wird im Vakuum für eine Stunde bei 7 Pa und Raumtemperatur getrocknet. 4.31 g eines viskosen, öligen Materials werden erhalten. Die Ausbeute an 1-Butyl-1-methylpyrrolidinium-tris(pentafluoroethyl)difluorochlorophosphat beträgt 92.4 %. Die Verbindung wird NMR-spektroskopisch analysiert.

NMR-Daten:
¹H NMR-Spektrum, ppm: 0.95 t (CH₃), 1.36 t,q (CH₂), 1.71 m (CH₂), 2.14 m (2CH₂), 2.93 s (CH₃), 3.22 m (CH₂), 3.40 m (2CH₂); J³_{H,H} = 7.4 Hz.
¹⁹F NMR-Spektrum, ppm: -24.42 d,m (PF), -67.12 d,m (PF), -79.57 m (3CF₃), -102.35 d (CF₂, F_{A}), -103.12 d (CF₂, F_{B}), -105.71 d,m (CF₂), -111.98 d,m (CF₂, F_{A}), -112.76 d,m (CF₂, F_{B}), -120.98 t (CF₂), -124.11 m (CF₂), - 124.29 m (CF₂), J¹_{P,F} = 937 Hz, J¹_{P,F} = 882 Hz, J²_{P,F} = 101 Hz, J²_{P,F} = 82 Hz, J²_{P,F} = 119 Hz, J²_{A,B} = 287 Hz.
³¹P NMR-Spektrum, ppm: -139.3 d,d,m.

### Beispiel 8: Tetramethylammonium-tris(pentafluoroethyl)difluorochlorophosphat

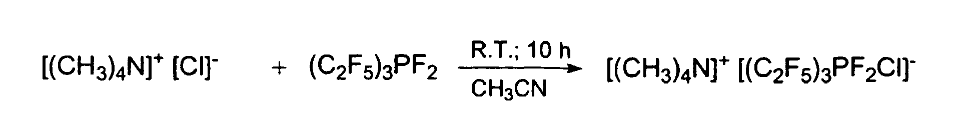

Eine Mischung aus 0.68 g (6.20 mmol) Tetramethylammoniumchlorid und 2.65 g (6.22 mmol) Tris(pentafluoroethyl)difluorophosphoran werden in 5 ml Acetonitril gelöst und bei Raumtemperatur für zehn Stunden gerührt. Flüchtige Bestandteile (Überschüsse an Tris(pentafluoroethyl)-difluorophosphoran und Acetonitril) werden im Vakuum entfernt und der Rückstand wird im Vakuum für 0.5 Stunden bei 7 Pa und Raumtemperatur getrocknet. 3.26 g eines festen Materials werden erhalten. Die Ausbeute an Tetramethylammoniumtris(pentafluoroethyl)difluorochlorophosphat beträgt 98.2 %, bezogen auf das eingesetzte Tetramethylammoniumchlorid. Die Verbindung (drei verschiedene Isomere) wird NMR-spektroskopisch analysiert.

NMR-Daten:
¹H NMR-Spektrum, ppm: 3.07 s (4CH₃).
¹⁹F NMR-Spektrum (facial Isomer), ppm: -68.9 br.d (PF₂), -78.25 m (2CF₃), -78.85 m (CF₃), -106.2 br.m (CF₂), -112.4 m (2CF₂), J¹_{P,F} = 960 Hz.
¹⁹F NMR-Spektrum (meridional Isomer 1), ppm: -22.44 d,m (PF₂), -78.2 m (2CF₃), -81.3 m (CF₃), -106.2 br.m (CF₂), -108.0 d,m (2CF₂), J¹_{P,F} = 873 Hz, J²_{P,F} = 85 Hz.
¹⁹F NMR-Spektrum (meridional Isomer 2), ppm: -25.50 d,m (PF), -69.83 d,m (PF), -77.48 t (CF₃), -80.03 m (2CF₃), -105.12 d,d (CF₂, F_{A}), -105.87 d,d (CF₂, F_{B}), -108.87 d,m (CF₂), -114.69 d,m (CF₂, F_{A}), -115.42 d,m (CF₂, F_{B}), J¹_{P,F} = 928 Hz, J¹_{P,F} = 861 Hz, J²_{P,F} = 100 Hz, J²_{P,F} = 82 Hz, J²_{P,F} = 116 Hz, J²_{A,B} = 282 Hz, J⁴_{F,F} = 22 Hz,.
³¹P NMR-Spektrum, ppm: -137.0 t,m (facial Isomer), -147.3 d,d,m (meridional Isomer 2), -149.9 t,m (meridional Isomer 1).

### Beispiel 9: 1-Butyl-1-methylpyrrolidinium-tris(pentafluoroethyl)difluorochlorophosphat

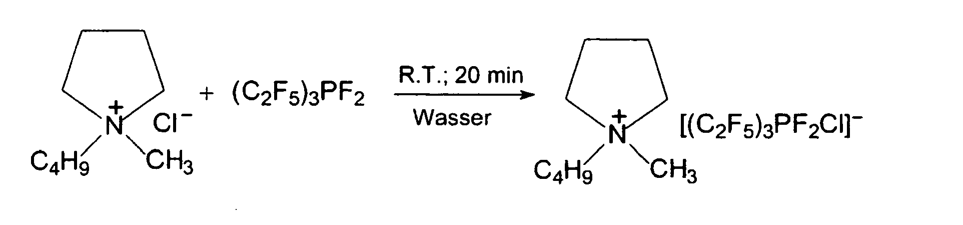

0.60 g (3.38 mmol) 1-Butyl-1-methylpyrrolidiniumchlorid Chlorid werden in 0.61 g Wasser bei Raumtemperatur aufgelöst. Zu dieser Lösung werden 1.44 g (3.38 mmol) Tris(pentafluoroethyl)difluorophosphoran bei Raumtemperatur innerhalb einer Minute dazugetropft. Das Reaktionsgemisch wird 20 min bei Raumtemperatur gerührt. Es bildet sich eine klare untere Phase des flüssigen Produkts, 1-Butyl-1-methylpyrrolidinium-tris(pentafluoroethyl)difluorochlorophosphat, die abgetrennt (1.77 g) und spektroskopisch untersucht wird.

NMR-Daten :
¹⁹F NMR-Spektrum (facial Isomer), Lösungsmittel - Wasser, elektronischer Standard (CCl₃F), ppm: -80.88 br.s, (CF₃), -82.15 br.s, (2CF₃), -87.09 br.d (PF₂), -114.71 br.d (3CF₂), J²_{P,F} = 87 Hz, J¹_{P,F} = 845 Hz.
³¹P NMR-Spektrum (facial Isomer), Lösungsmittel - Wasser, elektronischer Standard (H₃PO₄), ppm: -147.7 t,hep.

### Beispiel 10: 1-Ethyl-3-methylimidazolium-bis(pentafluoroethyl)phosphinat

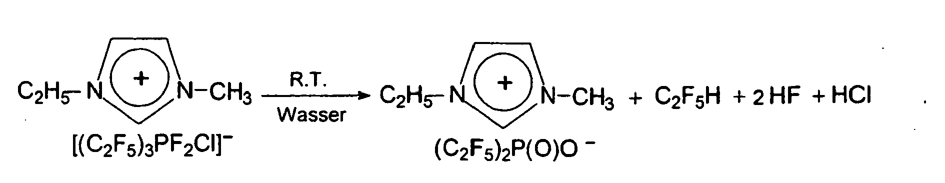

0.21 g (0.36 mmol) 1-Ethyl-3-methylimidazolium-bis(pentafluoroethyl)fluorochlorophosphat werden mit 2.0 g Wasser vermischt. Nach ca. 20 min intensiven Rührens ist das Gemisch homogen. Danach werden alle flüchtigen Bestandteile im Hochvakuum entfernt. Als Rest bleiben 0.15 g Öl, das spektroskopisch untersucht wird. Das ¹⁹F NMR-Spektrum und das ³¹P NMR-Spektrum zeigen die Bildung von 1-Ethyl-3-methylimidazolium-bis(pentafluoroethyl)phosphinat.

NMR-Daten :
¹⁹F NMR-Spektrum, Lösungsmittel - Wasser, elektronischer Standard (CCl₃F), ppm: -81.26 s, (2CF₃), -126.42 d (2CF₂), J²_{P,F} = 77 Hz

## Patentansprüche

1. Verbindung enthaltend Organofluorochlorophosphatanionen.

2. Verbindung gemäß Anspruch 1, **dadurch gekennzeichnet, dass** die Verbindung der Formel (I) entspricht
[Kt]^{z+} z[(CₙHₘF₂ₙ₊₁₋ₘ)xPCl_{y}F_{6-x-y}]⁻ (I)
worin [Kt]^{Z+} ein anorganisches oder organisches Kation bedeutet,
mit n = 1-12, m = 0 bis 2n+1, x = 1-4, y = 1-4, z = 1-4 und mit der Maßgabe dass x + y ≤ 5 ist.

3. Verbindung gemäß Anspruch 2, **dadurch gekennzeichnet, dass** m = 0 und y = 1 ist.

4. Verbindung gemäß Anspruch 2 oder 3, **dadurch gekennzeichnet, dass** x = 3 ist.

5. Verbindung gemäß einem oder mehreren der Ansprüche 2 bis 4, **dadurch gekennzeichnet, dass** n = 2, 3 oder 4 ist.

6. Verbindung gemäß einem oder mehreren der Ansprüche 2 bis 5, **dadurch gekennzeichnet, dass** die Verbindung der Formel (I) ausgewählt ist aus der Gruppe umfassend [Kt]^{z+} z[(C₂F₅)₃PClF₂]⁻, [Kt]^{z+} z[(C₃F₇)₃PCIF₂]⁻ oder [Kt]^{Z+} z[(C₄F₉)₃PClF₂]⁻.

7. Verbindung gemäß einem oder mehreren der Ansprüche 2 bis 6, **dadurch gekennzeichnet, dass** [Kt]^{z+} ein organisches Kation ist, das ausgewählt ist aus der Gruppe umfassend Ammonium-, Phosphonium, Uronium-, Thiouronium-, Guanidiniumkationen oder heterocyclische Kationen.

8. Verbindung gemäß einem oder mehreren der Ansprüche 2 bis 6,
**dadurch gekennzeichnet, dass** [Kt]^{z+} ein Ammoniumkation ist, das der Formel (1) entspricht,
[NR₄]⁺ (1),
wobei
R jeweils unabhängig voneinander
H, OR', NR'₂, mit der Maßgabe, dass maximal ein Substituent R in Formel (1) OR', NR'₂ ist,
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann,
wobei ein oder mehrere R teilweise oder vollständig mit Halogenen, insbesondere -F und/oder -Cl, oder teilweise mit -OH, -OR', -CN, -NR'₂, -C(O)OH, -C(O)NR'₂, -SO₂NR'₂, -C(O)X, -SO₂OH, -SO₂X, -NO₂, -SR', - S(O)R', -SO₂R', substituiert sein können, und wobei ein oder zwei nicht benachbarte und nicht α-ständige Kohlenstoffatome des R, durch Atome und/oder Atomgruppierungen ausgewählt aus der Gruppe -O-, -S-, - S(O)-, -SO₂-, -SO₂O-, -C(O)-. -C(O)O-, -N⁺R'₂-, -P(O)R'O-, -C(O)NR'-, - SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen sein kann.

9. Verbindung gemäß einem oder mehreren der Ansprüche 2 bis 6,
**dadurch gekennzeichnet, dass** [Kt]^{z+} ein Phosphoniumkation ist, das der Formel (2) entspricht
[PR²ₐ]⁺ (2),
wobei
R² jeweils unabhängig voneinander
H, OR' oder NR'₂
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere R² teilweise oder vollständig mit Halogenen, insbesondere -F und/oder -Cl, oder teilweise mit -OH, -OR', -CN, -NR'₂, -C(O)OH, -C(O)NR'₂, -SO₂NR'₂, -C(O)X, -SO₂OH, -SO₂X, -NO₂, -SR', - S(O)R', -SO₂R', substituiert sein können, und wobei ein oder zwei nicht benachbarte und nicht α-ständige Kohlenstoffatome des R², durch Atome und/oder Atomgruppierungen ausgewählt aus der Gruppe -O-, - S-, -S(O)-, -SO₂-, -SO₂O-, -C(O)-, -C(O)O-, -N⁺R'₂-, -P(O)R'O-, - C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

10. Verbindung gemäß einem oder mehreren der Ansprüche 2 bis 6,
**dadurch gekennzeichnet, dass** [Kt]^{z+} ein Uroniumkation ist, das der Formel (3) entspricht,
[C(R³R⁴N)(OR⁵)(NR⁶R⁷)]⁺ (3),
wobei
R³ bis R⁷ jeweils unabhängig voneinander
H, wobei H für R⁵ ausgeschlossen wird,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -Cl, oder teilweise mit -OH, -OR', -NR'₂, -CN, -C(O)OH, -C(O)NR'₂,-SO₂NR'₂, -C(O)X, -SO₂OH, -SO₂X, -SR', -S(O)R', -SO₂R', -NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, -S(O)-, -SO₂-, -SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cyloalkyl, unsubstituiertes oder substituiertes Phenyl und X =Halogen.

11. Verbindung gemäß einem oder mehreren der Ansprüche 2 bis 6,
**dadurch gekennzeichnet, dass** [Kt]^{z+} ein Thiouroniumkation ist, das der Formel (4) entspricht,
[C(R³R⁴N)(SR⁵)(NR⁶R⁷)]⁺ (4),
wobei
R³ bis R⁷ jeweils unabhängig voneinander
H, wobei H für R⁵ ausgeschlossen wird,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -Cl, oder teilweise mit -OH, -OR', -NR'₂, -CN, -C(O)OH, -C(O)NR'₂,-SO₂NR'₂, -C(O)X, -SO₂OH, -SO₂X, -SR', -S(O)R', -SO₂R', -NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, -S(O)-, -SO₂-, -SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

12. Verbindung gemäß einem oder mehreren der Ansprüche 2 bis 6,
**dadurch gekennzeichnet, dass** [Kt]^{z+} ein Guanidiniumkation ist, das der Formel (5) entspricht,
[C(NR⁸R⁹)(NR¹⁰R¹¹)(NR¹²R¹³)]⁺ (5),
wobei
R⁸ bis R¹³ jeweils unabhängig voneinander
H, -CN, NR'₂, -OR'
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R⁸ bis R¹³ teilweise oder vollständig mit Halogenen, insbesondere -F und/oder -Cl, oder teilweise mit -OH, -OR', -NR'₂, -CN, -C(O)OH, -C(O)NR'₂, - SO₂NR'₂, -C(O)X, -SO₂OH, -SO₂X, -SR', -S(O)R', -SO₂R', -NO₂, substituiert sein können, und wobei ein oder zwei nicht benachbarte und nicht α-ständige Kohlenstoffatome von R⁸ bis R¹³ durch Atome und/oder Atomgruppierungen ausgewählt aus der Gruppe -O-, -S-, -S(O)-, -SO₂-, -SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

13. Verbindung gemäß einem oder mehreren der Ansprüche 2 bis 6,
**dadurch gekennzeichnet, dass** [Kt]^{z+} der Formel (6) entspricht,
[HetN]^{z+} (6)
wobei
HetN^{z+} ein heterocyclisches Kation, ausgewählt aus der Gruppe 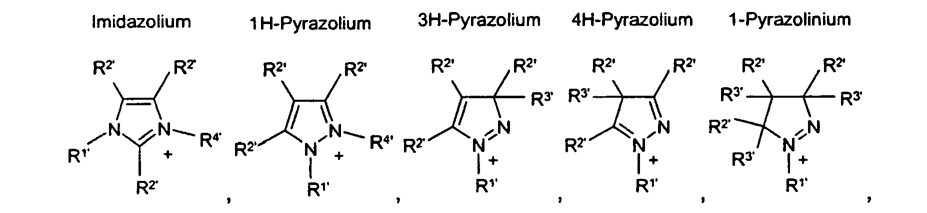 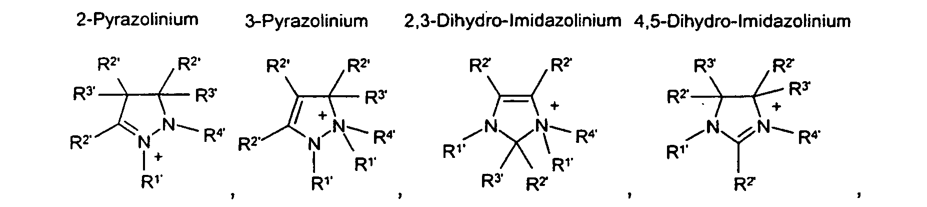 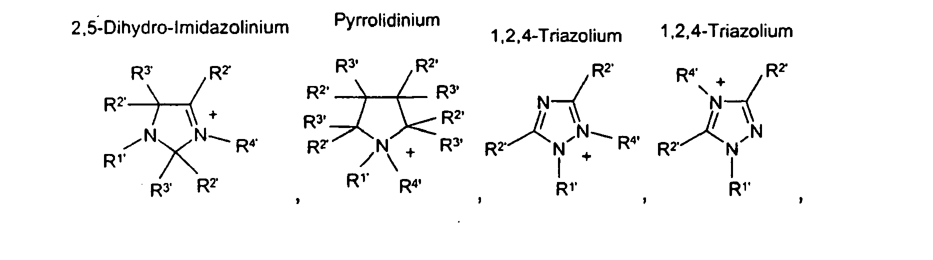 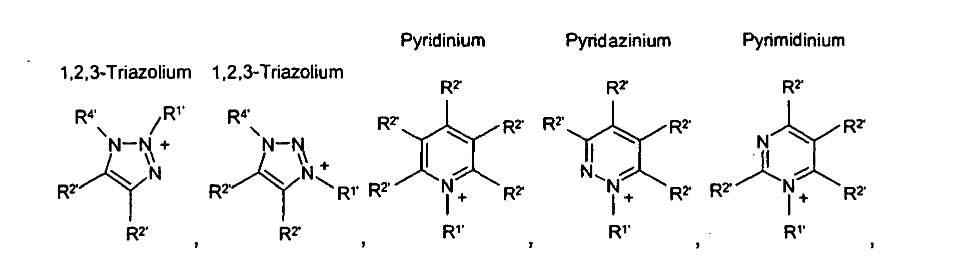 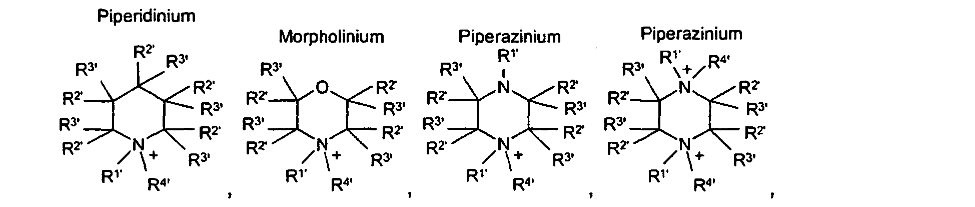 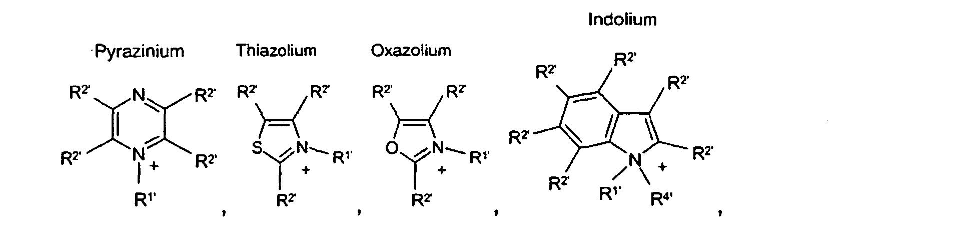 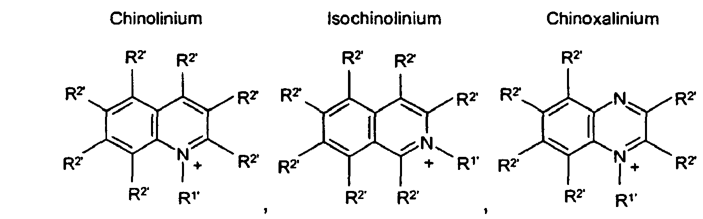 oder 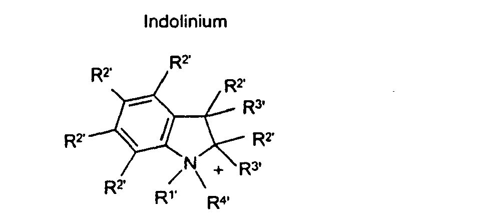
bedeutet, wobei die Substituenten
R¹_{,} bis R⁴_{,} jeweils unabhängig voneinander
H, F, Cl, Br, J, -CN, -OR', -NR'₂, -P(O)R'₂, -P(O)(OR')₂, -P(O)(NR'₂)₂, - C(O)R', -C(O)OR', -C(O)X, -C(O)NR'₂, -SO₂NR'₂, -SO₂OH, -SO₂X, -SR', -S(O)R', -SO₂R' und/oder NO₂ bedeuten, mit der Maßgabe, dass R¹', R³', R⁴' gleich H und/oder ein geradkettiges oder verzweigtes Alkyl mit 1-20 C-Atomen,
geradkettiges oder verzweigtes Alkenyl mit 2-20 C-Atomen und einer oder mehreren Doppelbindungen ist,
geradkettiges oder verzweigtes Alkyl mit 1-20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl bedeutet,
wobei die Substituenten R^{1'}, R^{2'} , R^{3'} und/oder R^{4'} zusammen auch ein Ringsystem bilden können,
wobei ein oder mehrere Substituenten R¹' bis R⁴' teilweise oder vollständig mit Halogenen, insbesondere -F und/oder -Cl, oder -OH, - OR', NR'₂ , -CN, -C(O)OH, -C(O)NR'₂, -SO₂NR'₂, -C(O)X, -SO₂OH, - SO₂X, -SR', -S(O)R', -SO₂R', -NO₂, substituiert sein können, wobei jedoch nicht gleichzeitig R^{1'} und R^{4'} vollständig mit Halogenen substituiert sein dürfen, und wobei ein oder zwei nicht benachbarte und nicht am Heteroatom gebundene Kohlenstoffatome der Substituenten R^{1,} bis R^{4,}, durch Atome und/oder Atomgruppierungen ausgewählt aus der -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₁₈-Alkyl, C₃- bis C₇-Cycloalkyl, unsubstituiertes oder substituiertes Phenyl und X =Halogen.

14. Verbindung gemäß einem oder mehreren der Ansprüche 2 bis 6,
**dadurch gekennzeichnet, dass** [Kt]^{z+} ein [(R°)₃O]⁺-Kation oder ein [(R°)₃S]⁺-Kation ist, wobei R° geradkettige oder verzweigte Alkylgruppen mit 1-8 C-Atomen oder unsubstituiertes Phenyl oder mit R°, OR°, N(R°)₂, CN oder Halogen substituiertes Phenyl bedeutet.

15. Verbindung gemäß einem oder mehreren der Ansprüche 2 bis 6,
**dadurch gekennzeichnet, dass** [Kt]^{z+} ein Metallkation ist.

16. Verbindung gemäß Anspruch 15, **dadurch gekennzeichnet, dass** das Metallkation ein Metall der Gruppen 1 bis 12 des Periodensystems umfasst.

17. Verbindung gemäß Anspruch 15 oder 16, **dadurch gekennzeichnet,**
**dass** das Metall ausgewählt ist aus der Gruppe umfassend Li, Na, K, Rb, Cs.

18. Verbindung gemäß einem oder mehreren der Ansprüche 1 bis 13,
**dadurch gekennzeichnet, dass** es sich um Imidazolium-, Pyridinium-, Pyrrolidinium-, Ammonium-, Phosphonium-, Guanidinium-Salze mit [(C₂F₅)₃PClF₂]⁻-, [(C₃F₇)₃PClF₂]⁻- oder [(C₄F₉)₃PClF₂]⁻ -Anionen handelt.

19. Verfahren zur Herstellung von Verbindungen mit Organofluorochlorophosphatanionen umfassend die Umsetzung eines Chloridsalzes mit Organofluorophosporanen.

20. Verfahren zur Herstellung einer Verbindung (I) gemäß einem oder mehreren der Ansprüche 2 bis 18 umfassend die Umsetzung von [Kt]^{z+} z[Cl]⁻ mit einer Verbindung der allgemeinen Formel (II)
(CₙHₘF₂ₙ₊₁₋ₘ)ₓPF₅₋ₓ (II)
worin [Kt]^{z+} ein anorganisches oder organisches Kation mit z = 1-4 und in den Verbindungen der Formel II n = 1-12, m = 0 bis 2n+1, x = 1-4 und y = 1-4 bedeutet.

21. Verfahren gemäß Anspruch 20, **dadurch gekennzeichnet, dass** in Verbindungen der Formel II y = 1 und m = 0 ist.

22. Verfahren gemäß Anspruch 20 oder 21, **dadurch gekennzeichnet, dass** die Umsetzung bei Temperaturen von -40 bis 180 °C erfolgt.

23. Verfahren gemäß einem oder mehreren der Ansprüche 20 bis 22,
**dadurch gekennzeichnet, dass** die Umsetzung in einem Lösungsmittel erfolgt.

24. Verfahren gemäß Anspruch 23, **dadurch gekennzeichnet, dass** das Lösungsmittel ausgewählt ist aus der Gruppe der Nitrile, Dialkylcarbonate, Glymes, Dialkylethers, Cyclic Ethers, Dimethylfomamide, Dimethytsulfoxide, Dichlormethane, Wasser oder Mischungen hieraus.

25. Verwendung einer Verbindung gemäß einem oder mehreren der Ansprüche 1 bis 18 zur Herstellung von Organophosphinaten.

26. Verfahren zur Herstellung von Organophosphinaten umfassend die Umsetzung einer Verbindung gemäß einem oder mehreren der Ansprüche 1 bis 18 mit Wasser oder Wasser enthaltenden Lösungsmitteln oder Lösungsmittelgemischen.

27. Verfahren gemäß Anspruch 26, **dadurch gekennzeichnet, dass** die Umsetzung bei -40 bis 200°C erfolgt.

28. Verwendung einer Verbindung gemäß einem oder mehreren der Ansprüche 1 bis 18 als Lösungsmittel oder Lösungsmittelzusatz, als Phasentransferkatalysator, als Extraktionsmittel, als Wärmeträger, als oberflächenaktive Substanz, als Weichmacher, als Flammschutzmittel, als Additiv oder als Leitsalz und Katalysatoren in chemischen Prozessen.

29. Elektrolyte, elektrochemische Zellen, Kondensatoren oder Brennstoffzellen enthaltend mindestens eine Verbindung gemäß einem oder mehreren der Ansprüche 1 bis 18.

## Claims

1. Compound containing organofluorochlorophosphate anions.

2. Compound according to Claim 1, **characterised in that** the compound conforms to the formula (I)
[Kt]^{z+} z[(CₙHₘF₂ₙ₊₁₋ₘ)ₓPCl_{y}F_{6-x-y}]⁻ (I)
in which [Kt]^{z+} denotes an inorganic or organic cation, where n = 1-12, m = 0 to 2n+1, x = 1-4, y = 1-4, z = 1-4 and with the proviso that x + y is ≤ 5.

3. Compound according to Claim 2, **characterised in that** m = 0 and y = 1.

4. Compound according to Claim 2 or 3, **characterised in that** x = 3.

5. Compound according to one or more of Claims 2 to 4, **characterised in that** n = 2, 3 or 4.

6. Compound according to one or more of Claims 2 to 5, **characterised in that** the compound of the formula (I) is selected from the group comprising [Kt]^{z+} z[(C₂F₅)₃PClF₂]⁻, [Kt]^{z+} z[(C₃F₇)₃PClF₂]⁻ or [Kt]^{z+} z[(C₄F₉)₃PClF₂]⁻.

7. Compound according to one or more of Claims 2 to 6, **characterised in that** [Kt]^{z+} is an organic cation which is selected from the group comprising ammonium, phosphonium, uronium, thiouronium, guanidinium cations or heterocyclic cations.

8. Compound according to one or more of Claims 2 to 6, **characterised in that** [Kt]^{z+} is an ammonium cation, which conforms to the formula (1)
[NR₄]⁺ (1),
where
R in each case, independently of one another, denotes H, OR', NR'₂, with the proviso that a maximum of one substituent R in formula (1) is OR', NR'₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially or fully substituted by halogens, in particular -F and/or -Cl, or partially by -OH, -OR', -CN, -NR'₂, -C(O)OH, -C(O)NR'₂, -SO₂NR'₂, -C(O)X, -SO₂OH, -SO₂X, -NO₂, -SR', -S(O)R', -SO₂R', and where one or two non-adjacent carbon atoms in R which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' may be = H, non-, partially or perfluorinated C₁- to C₁₈-alkyl, C₃- to C₇-cycloalkyl, unsubstituted or substituted phenyl and X may be = halogen.

9. Compound according to one or more of Claims 2 to 6, **characterised in that** [Kt]^{z+} is a phosphonium cation, which conforms to the formula (2)
[PR²₄]⁺ (2),
where
R² in each case, independently of one another, denotes H, OR' or NR'₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds, saturated, partially or fully unsaturated cycloalkyl having 3-7 C atoms, which may be substituted by alkyl groups having 1-6 C atoms, where one or more R² may be partially or fully substituted by halogens, in particular -F and/or -Cl, or partially by -OH, -OR', -CN, -NR'₂, -C(O)OH, -C(O)NR'₂, -SO₂NR'₂, -C(O)X, -SO₂OH, -SO₂X, -NO₂, -SR', -S(O)R', -SO₂R', and where one or two non-adjacent carbon atoms in R² which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' = H, non-, partially or perfluorinated C₁- to C₁₈-alkyl, C₃- to C₇-cycloalkyl, unsubstituted or substituted phenyl and X = halogen.

10. Compound according to one or more of Claims 2 to 6, **characterised in that** [Kt]^{z+} is a uronium cation, which conforms to the formula (3)
[C(R³R⁴N)(OR⁵)(NR⁶R⁷)]⁺ (3),
where
R³ to R⁷ each, independently of one another, denote H, where H is excluded for R⁵,
straight-chain or branched alkyl having 1 to 20 C atoms, straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R³ to R⁷ may be partially or fully substituted by halogens, in particular -F and/or -Cl, or partially by -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, and where one or two non-adjacent carbon atoms in R³ to R⁷ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' = H, non-, partially or perfluorinated C₁- to C₁₈-alkyl, C₃- to C₇-cycloalkyl, unsubstituted or substituted phenyl and X = halogen.

11. Compound according to one or more of Claims 2 to 6, **characterised in that** [Kt]^{z+} is a thiouronium cation, which conforms to the formula (4)
[C(R³R⁴N)(SR⁵)(NR⁶R⁷)]⁺ (4),
where
R³ to R⁷ each, independently of one another, denote H, where H is excluded for R⁵,
straight-chain or branched alkyl having 1 to 20 C atoms, straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R³ to R⁷ may be partially or fully substituted by halogens, in particular -F and/or -Cl, or partially by -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, and where one or two non-adjacent carbon atoms in R³ to R⁷ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' = H, non-, partially or perfluorinated C₁- to C₁₈-alkyl, C₃- to C₇-cycloalkyl, unsubstituted or substituted phenyl and X = halogen.

12. Compound according to one or more of Claims 2 to 6, **characterised in that** [Kt]^{z+} is a guanidinium cation, which conforms to the formula (5)
[C(NR⁸R⁹)(NR¹⁰R¹¹)(NR¹²R¹³)]⁺ (5),
where
R⁸ to R¹³ each, independently of one another, denote H, -CN, NR'₂, -OR',
straight-chain or branched alkyl having 1 to 20 C atoms, straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R⁸ to R¹³ may be partially or fully substituted by halogens, in particular -F and/or -Cl, or partially by -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, and where one or two non-adjacent carbon atoms in R⁸ to R¹³ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' = H, non-, partially or perfluorinated C₁- to C₁₈-alkyl, C₃- to C₇-cycloalkyl, unsubstituted or substituted phenyl and
X = halogen.

13. Compound according to one or more of Claims 2 to 6, **characterised in that** [Kt]^{z+} conforms to the formula (6)
[HetN]^{z+} (6)
where
HetN^{z+} denotes a heterocyclic cation selected from the group 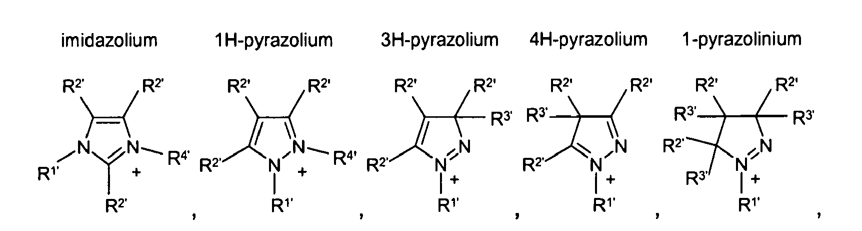 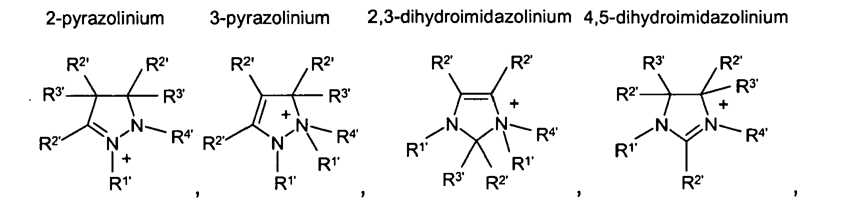 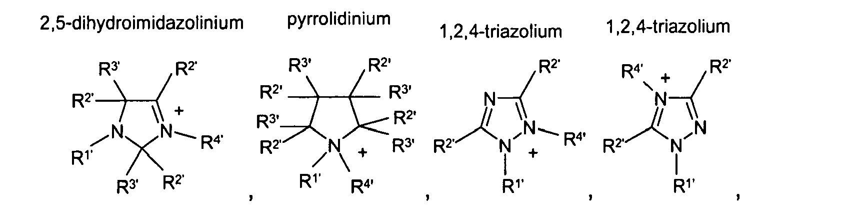 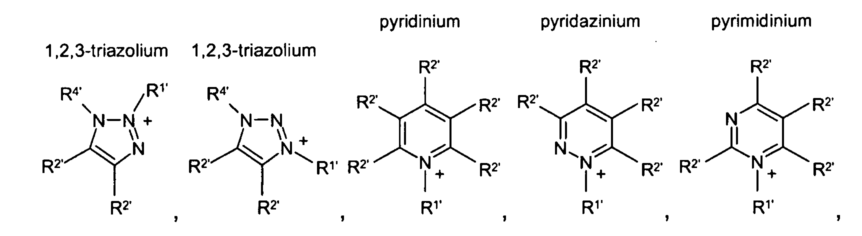 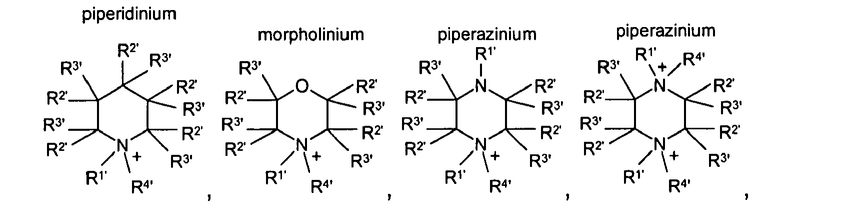 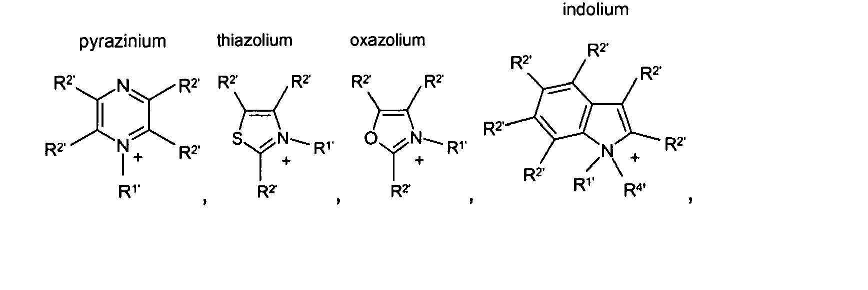 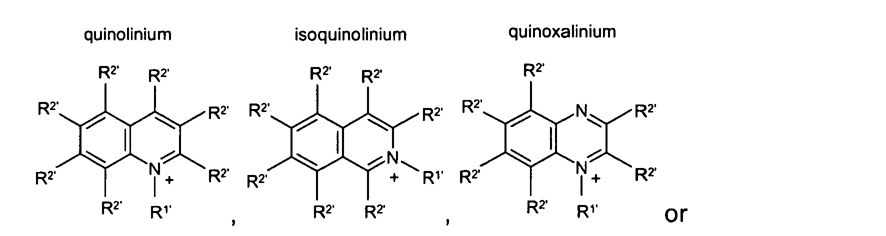 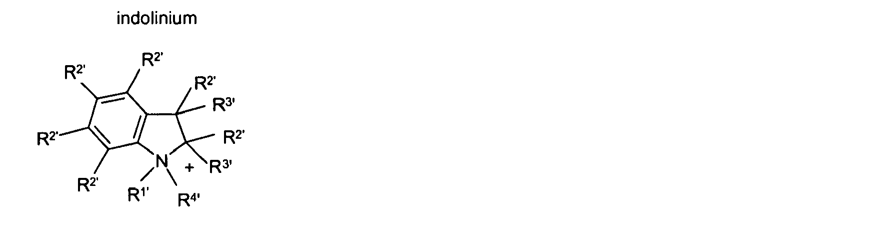
where the substituents
R^{1,} to R^{4,} each, independently of one another, denote H, F, Cl, Br, I, -CN, -OR', -NR'₂, -P(O)R'₂, -P(O)(OR')₂, -P(O)(NR'₂)₂, -C(O)R', -C(O)OR', -C(O)X, -C(O)NR'₂, -SO₂NR'₂, -SO₂OH, -SO₂X, -SR', -S(O)R', -SO₂R' and/or NO₂, with the proviso that R¹', R³', R⁴' are H and/or a straight-chain or branched alkyl having 1-20 C atoms, straight-chain or branched alkenyl having 2-20 C atoms and one or more double bonds,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, saturated, partially or fully unsaturated heteroaryl, heteroaryl-C₁-C₆-alkyl or aryl-C₁-C₆-alkyl,
where the substituents R^{1'}, R^{2'} , R^{3'} and/or R^{4'} together may also form a ring system,
where one or more substituents R¹' to R⁴' may be partially or fully substituted by halogens, in particular -F and/or -Cl, or -OH, -OR', NR'₂, -CN, -C(O)OH, -C(O)NR'₂, -SO₂NR'₂, -C(O)X, -SO₂OH, -SO₂X, -SR', -S(O)R', -SO₂R', -NO₂, but where R¹' and R⁴' cannot simultaneously be fully substituted by halogens, and where, in the substituents R¹' to R⁴', one or two non-adjacent carbon atoms which are not bonded to the heteroatom may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'-, where R' = H, non-, partially or perfluorinated C₁- to C₁₈-alkyl, C₃- to C₇-cycloalkyl, unsubstituted or substituted phenyl and X = halogen.

14. Compound according to one or more of Claims 2 to 6, **characterised in that** [Kt]^{z+} is a [(R°)₃O]⁺ cation or a [(R°)₃S]⁺ cation, where R° denotes straight-chain or branched alkyl groups having 1-8 C atoms or unsubstituted phenyl or phenyl which is substituted by R°, OR°, N(R°)₂, CN or halogen.

15. Compound according to one or more of Claims 2 to 6, **characterised in that** [Kt]^{z+} is a metal cation.

16. Compound according to Claim 15, **characterised in that** the metal cation comprises a metal from groups 1 to 12 of the Periodic Table.

17. Compound according to Claim 15 or 16, **characterised in that** the metal is selected from the group comprising Li, Na, K, Rb, Cs.

18. Compound according to one or more of Claims 1 to 13, **characterised in that** it is an imidazolium, pyridinium, pyrrolidinium, ammonium, phosphonium or guanidinium salt containing a [(C₂F₅)₃PClF₂]⁻, [(C₃F₇)₃PClF₂]⁻ or [(C₄F₉)₃PClF₂]⁻ anion.

19. Process for the preparation of compounds containing organofluorochlorophosphate anions comprising the reaction of a chloride salt with organofluorophosphoranes.

20. Process for the preparation of a compound (I) according to one or more of Claims 2 to 18 comprising the reaction of [Kt]^{z+} z[Cl]⁻ with a compound of the general formula (II)
(CₙHₘF₂ₙ₊₁₋ₘ)ₓPF₅₋ₓ (II)
in which [Kt]^{z+} denotes an inorganic or organic cation where z = 1-4, and in the compounds of the formula II n = 1-12, m = 0 to 2n+1, x = 1-4 and y = 1-4.

21. Process according to Claim 20, **characterised in that** in compounds of the formula II y = 1 and m = 0.

22. Process according to Claim 20 or 21, **characterised in that** the reaction is carried out at temperatures of -40 to 180°C.

23. Process according to one or more of Claims 20 to 22, **characterised in that** the reaction is carried out in a solvent.

24. Process according to Claim 23, **characterised in that** the solvent is selected from the group of the nitriles, dialkyl carbonates, glymes, dialkyl ethers, cyclic ethers, dimethylformamide, dimethyl sulfoxide, dichloromethane, water or mixtures thereof.

25. Use of a compound according to one or more of Claims 1 to 18 for the preparation of organophosphinates.

26. Process for the preparation of organophosphinates comprising the reaction of a compound according to one or more of Claims 1 to 18 with water or water-containing solvents or solvent mixtures.

27. Process according to Claim 26, **characterised in that** the reaction is carried out at -40 to 200°C.

28. Use of a compound according to one or more of Claims 1 to 18 as solvent or solvent additive, as phase-transfer catalyst, as extractant, as heat-transfer medium, as surface-active substance, as plasticiser, as flameproofing agent, as additive or as conductive salt and catalysts in chemical processes.

29. Electrolytes, electrochemical cells, capacitors or fuel cells comprising at least one compound according to one or more of Claims 1 to 18.

## Revendications

1. Composé contenant des anions d'organofluorochlorophosphate.

2. Composé selon la revendication 1, **caractérisé en ce que** le composé est conforme à la formule (I)
[Kt]^{z+} z[(CₙHₘF₂ₙ₊₁-ₘ)ₓPCl_{y}F_{6-x-y}]⁻ (I)
dans laquelle [Kt]^{z+} représente un cation inorganique ou organique, où n = 1-12, m =0 à2n+1, x = 1-4, y= 1-4, z= 1-4 et sous réserve que x + y est ≤ 5.

3. Composé selon la revendication 2, **caractérisé en ce que** m = 0 et y= 1.

4. Composé selon la revendication 2 ou 3, **caractérisé en ce que** x = 3.

5. Composé selon une ou plusieurs des revendications 2 à 4, **caractérisé en ce que** n = 2, 3 ou 4.

6. Composé selon une ou plusieurs des revendications 2 à 5, **caractérisé en ce que** le composé de la formule (I) est choisi parmi le groupe comprenant [Kt]^{z+} z[(C₂F₅)₃PClF₂]⁻, [Kt]^{z+} z[(C₃F₇)₃PClF₂]⁻ ou [Kt]^{z+} z[(C₄F₉)₃PClF₂]⁻.

7. Composé selon une ou plusieurs des revendications 2 à 6, **caractérisé en ce que** [Kt]^{z+} est un cation organique qui est choisi parmi le groupe comprenant des cations ammonium, phosphonium, uronium, thiouronium, guanidinium ou des cations hétérocycliques.

8. Composé selon une ou plusieurs des revendications 2 à 6, **caractérisé en ce que** [Kt]^{z+} est un cation ammonium, lequel est conforme à la formule (1)
[NR₄]⁺ (1),
où
R dans chaque cas indépendamment les uns des autres, représente H, OR', NR'₂, sous réserve qu'un maximum d'un substituant R dans la formule (1) est OR', NR'₂,
alkyle en chaîne droite ou ramifié comportant 1-20 atomes de C, alkényle en chaîne droite ou ramifié comportant 2-20 atomes de C et une ou plusieurs liaisons doubles,
alkynyle en chaîne droite ou ramifié comportant 2-20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C,
où un ou plusieurs R peuvent être partiellement ou complètement substitués par halogènes, en particulier -F et/ou -Cl, ou partiellement substitués par -OH, -OR', -CN, -NR'₂, -C(O)OH, -C(O)NR'₂, -SO₂NR'₂, -C(O)X, -SO₂OH, -SO₂X, -NO₂, -SR', -S(O)R', -SO₂R', et où un ou deux atomes de carbone non adjacents dans R, qui ne sont pas à la position
α, peuvent être remplacés par des atomes et/ou des groupes d'atomes choisis parmi le groupe -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' peut être = H, C₁- à C₁₈-alkyle non perfluoré, partiellement perfluoré ou perfluoré, C₃- à C₇-, phényle non substitué ou substitué et X peut être = halogène.

9. Composé selon une ou plusieurs des revendications 2 à 6, **caractérisé en ce que** [Kt]^{z+} est un cation phosphonium, lequel est conforme à la formule (2)
[PR²₄]⁺ (2),
où
R² dans chaque cas indépendamment les uns des autres représente H, OR' ou NR'₂,
alkyle en chaîne droite ou ramifié comportant 1-20 atomes de C, alkényle en chaîne droite ou ramifié comportant 2-20 atomes de C et une ou plusieurs liaisons doubles,
alkynyle en chaîne droite ou ramifié comportant 2-20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C,
où un ou plusieurs R² peuvent être partiellement ou complètement substitués par halogènes, en particulier -F et/ou -Cl, ou partiellement substitués par -OH, -OR', -CN, -NR'₂, -C(O)OH, -C(O)NR'₂, -SO₂NR'₂, -C(O)X, -SO₂OH, -SO₂X, -NO₂, -SR', -S(O)R', -SO₂R', et où un ou deux atomes de carbone non adjacents dans R², lesquels ne sont pas à la position α, peuvent être remplacés par des atomes et/ou des groupes d'atomes choisis parmi le groupe constitué par -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' = H, C₁- à C₁₈-alkyle non perfluoré, partiellement perfluoré ou perfluoré, C₃- à C₇-cycloalkyle, phényle non substitué ou substitué et X = halogène.

10. Composé selon une ou plusieurs des revendications 2 à 6, **caractérisé en ce que** [Kt]^{z+} est un cation uronium, lequel est conforme à la formule (3)
[C(R³R⁴N)(OR⁵)(NR⁶R⁷)]⁺ (3),
où
R³ à R⁷ chacun indépendamment les uns des autres représentent H, où H est exclus pour R⁵,
alkyle en chaîne droite ou ramifié comportant de 1 à 20 atomes de C, alkényle en chaîne droite ou ramifié comportant de 2 à 20 atomes de C et une ou plusieurs liaisons doubles,
alkynyle en chaîne droite ou ramifié comportant de 2 à 20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C, où un ou plusieurs des substituants R³ à R⁷ peuvent être partiellement ou complètement substitués par halogènes, en particulier -F et/ou -Cl, ou partiellement substitués par -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, et où un ou deux atomes de carbone non adjacents dans R³ à R⁷, lesquels ne sont pas à la position α, peuvent être remplacés par des atomes et/ou des groupes d'atomes choisis parmi le groupe constitué par -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' = H, C₁- à C₁₈-alkyle non perfluoré, partiellement perfluoré ou perfluoré, C₃- à C₇-cycloalkyle, phényle non substitué ou substitué et X = halogène.

11. Composé selon une ou plusieurs des revendications 2 à 6, **caractérisé en ce que** [Kt]^{z+} est un cation thiouronium, qui est conforme à la formule (4)
[C(R³R⁴N)(SR⁵)(NR⁶R⁷)]⁺ (4),
où
R³ à R⁷ chacun indépendamment les uns des autres représentent H, où H est exclu pour R⁵,
alkyle en chaîne droite ou ramifié comportant de 1 à 20 atomes de C, alkényle en chaîne droite ou ramifié comportant 2-20 atomes de C et une ou plusieurs liaisons doubles,
alkynyle en chaîne droite ou ramifié comportant 2-20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C, où un ou plusieurs des substituants R³ à R⁷ peuvent être partiellement ou complètement substitués par halogènes, en particulier -F et/ou -Cl, ou partiellement substitués par -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, et où un ou deux atomes de carbone non adjacents dans R³ à R⁷, lesquels ne sont pas à la position α, peuvent être remplacés par des atomes et/ou des groupes d'atomes choisis parmi le groupe constitué par -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' = H, C₁- à C₁₈-alkyle non perfluoré, partiellement perfluoré ou perfluoré, C₃- à C₇-cycloalkyle, phényle non substitué ou substitué et X = halogène.

12. Composé selon une ou plusieurs des revendications 2 à 6, **caractérisé en ce que** [Kt]^{z+} est un cation guanidinium, qui est conforme à la formule (5)
[C(NR⁸R⁹)(NR¹⁰R¹¹)(NR¹²R¹³)]⁺ (5),
où
R⁸ à R¹³ chacun indépendamment les uns des autres représentent H, -CN, NR'₂, -OR',
alkyle en chaîne droite ou ramifié comportant de 1 à 20 atomes de C, alkényle en chaîne droite ou ramifié comportant de 2 à 20 atomes de C et une ou plusieurs liaisons doubles,
alkynyle en chaîne droite ou ramifié comportant de 2 à 20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C, où un ou plusieurs des substituants R⁸ à R¹³ peuvent être partiellement ou complètement substitués par halogènes, en particulier -F et/ou -Cl, ou partiellement substitués par -OH, -OR', -NR'₂, -CN, -C(O)OH, -C(O)NR'₂, -SO₂NR'₂, -C(O)X, -SO₂OH, -SO₂X, -SR', -S(O)R', -SO₂R', -NO₂, et où un ou deux atomes de carbone non adjacents dans R⁸ à R¹³, lesquels ne sont pas à la position α, peuvent être remplacés par des atomes et/ou des groupes d'atomes choisis parmi le groupe constitué par -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' = H, C₁- à C₁₈-alkyle non perfluoré, partiellement perfluoré ou perfluoré, C₃- à C₇-cycloalkyle, phényle non substitué ou substitué et X = halogène.

13. Composé selon une ou plusieurs des revendications 2 à 6, **caractérisé en ce que** [Kt]^{z+} est conforme à la formule (6)
[HetN]^{z+} (6)
où
HetN^{z+} représente un cation hétérocyclique choisi parmi le groupe constitué par 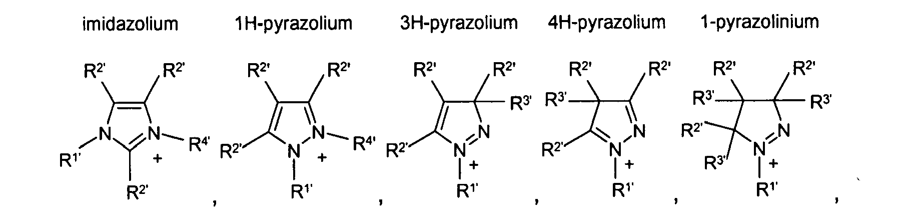 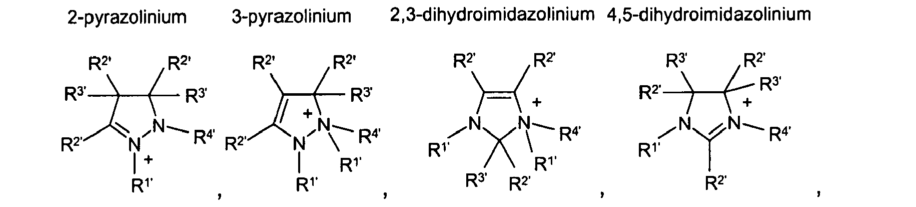 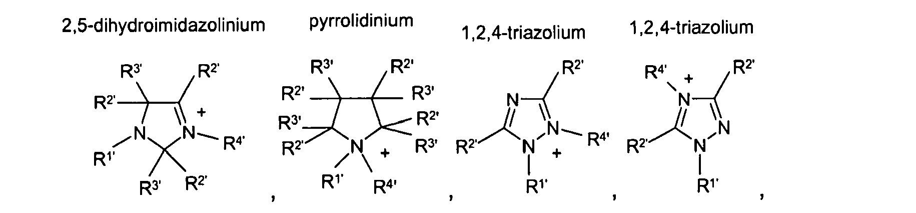 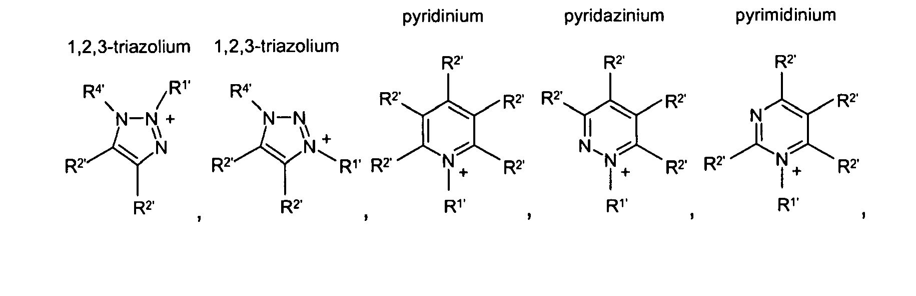 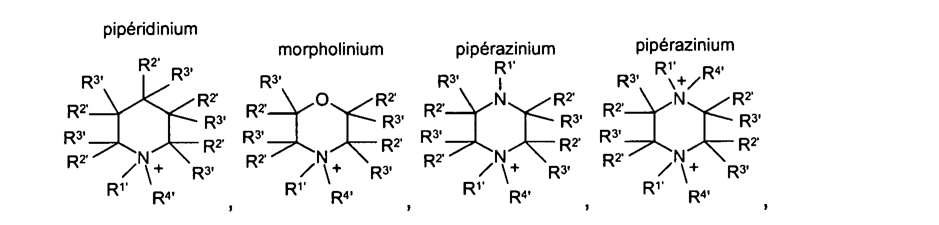 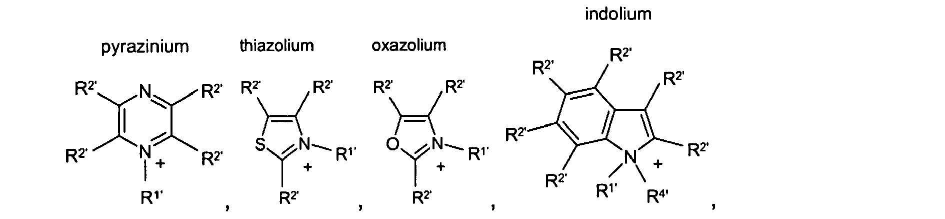 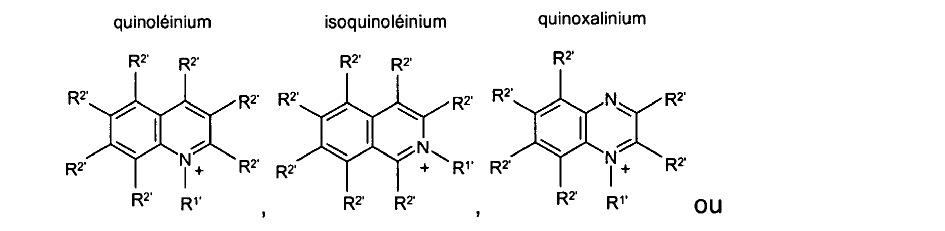 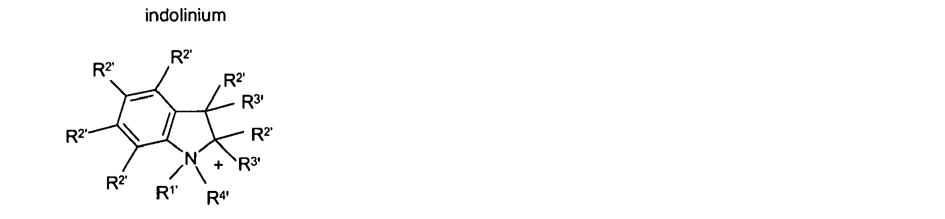
où les substituants
R^{1,} à R^{4,} chacun indépendamment les uns des autres représentent H, F, Cl, Br, l, -CN, -OR', -NR'₂, -P(O)R'₂, -P(O)(OR')₂, -P(O)(NR'₂)₂, -C(O)R', -C(O)OR', -C(O)X, -C(O)NR'₂, -SO₂NR'₂, -SO₂OH, -SO₂X, -SR', -S(O)R', -SO₂R' et/ou NO₂, sous réserve que R¹', R³', R⁴' sont H et/ou un alkyle en chaîne droite ou ramifié comportant 1-20 atomes de C, alkényle en chaîne droite ou ramifié comportant 2-20 atomes de C et une ou plusieurs liaisons doubles,
alkyle en chaîne droite ou ramifié comportant 1-20 atomes de C, alkényle en chaîne droite ou ramifié comportant 2-20 atomes de C et une ou plusieurs liaisons doubles, alkynyle en chaîne droite ou ramifié comportant 2-20 atomes de C et une ou plusieurs liaisons triples,
cycloalkyle saturé, partiellement ou complètement non saturé comportant 3-7 atomes de C, qui peut être substitué par des groupes alkyle comportant 1-6 atomes de C,
hétéroaryle saturé, partiellement ou complètement non saturé, hétéroaryle-C₁-C₆-alkyle ou aryle-C₁-C₆-alkyle,
où les substituants R^{1'}, R^{2'}, R^{3'} et/ou R^{4'} peuvent également former ensemble un système de cycle,
où un ou plusieurs substituants R¹' à R⁴' peuvent être partiellement ou complètement substitués par halogènes, en particulier -F et/ou -Cl, ou -OH, -OR', NR'₂, -CN, -C(O)OH, -C(O)NR'₂, -SO₂NR'₂, -C(O)X, -SO₂OH, -SO₂X, -SR', -S(O)R', -SO₂R', -NO₂, mais où R^{1'} et R^{4'} ne peuvent pas simultanément être complètement substitués par halogènes, et où, dans les substituants R¹' à R⁴' un ou deux atomes de carbone non adjacents qui ne sont pas liés à l'hétéroatome peuvent être remplacés par des atomes et/ou des groupes d'atomes choisis parmi le groupe -O-, -S-, -S(O)-, -SO-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- ou -P(O)R'-, où R' = H, C₁- à C₁₈-alkyle non perfluoré, partiellement perfluoré ou perfluoré, C₃- à C₇-cycloalkyle, phényle non substitué ou substitué et X = halogène.

14. Composé selon une ou plusieurs des revendications 2 à 6, **caractérisé en ce que** [Kt]^{z+} est un cation [(R°)₃O]⁺ ou un cation [(R°)₃S]⁺, où R° représente des groupes alkyle en chaîne droite ou ramifié comportant 1-8 atomes de C ou phényle non substitué ou phényle qui est substitué par R°, OR°, N(R°)₂, CN ou halogène.

15. Composé selon une ou plusieurs des revendications 2 à 6, **caractérisé en ce que** [Kt]^{z+} est un cation métallique.

16. Composé selon la revendication 15, **caractérisé en ce que** le cation métallique comprend un métal pris parmi les groupes 1 à 12 du Tableau Périodique.

17. Composé selon la revendication 15 ou 16, **caractérisé en ce que** le métal est choisi parmi le groupe comprenant Li, Na, K, Rb, Cs.

18. Composé selon une ou plusieurs des revendications 1 à 13, **caractérisé en ce qu'**il s'agit d'un sel d'imidazolium, de pyridinium, de pyrrolidinium, d'ammonium, de phosphonium ou de guanidinium contenant un anion [(C₂F₅)₃PClF₂]⁻, [(C₃F₇)₃PClF₂]⁻ ou [(C₄F₉)₃PClF₂]⁻.

19. Procédé pour la préparation de composés contenant des anions d'organofluorochlorophosphate comprenant la réaction d'un sel de chlorure avec des organofluorophosphoranes.

20. Procédé pour la préparation d'un composé (I) selon une ou plusieurs des revendications 2 à 18 comprenant la réaction de [Kt]^{z+} z[CI]⁻ avec un composé de la formule générale (II)
(CₙHₘF₂ₙ₊₁₋ₘ)ₓPF₅₋ₓ (II)
dans laquelle [Kt]^{z+} représente un cation inorganique ou organique où z = 1-4, et dans les composés de la formule II, n = 1-12, m = 0 à 2n+1, x=1-4ety=1-4.

21. Procédé selon la revendication 20, **caractérisé en ce que**, dans les composés de la formule II, y = 1 et m = 0.

22. Procédé selon la revendication 20 ou 21, **caractérisé en ce que** la réaction est mise en oeuvre à des températures de -40 à 180°C.

23. Procédé selon une ou plusieurs des revendications 20 à 22, **caractérisé en ce que** la réaction est mise en oeuvre dans un solvant.

24. Procédé selon la revendication 23, **caractérisé en ce que** le solvant est choisi parmi le groupe nitriles, carbonates de dialkyle, glymes, éthers de dialkyle, éthers cycliques, diméthylformamide, sulfoxyde de di-méthyle, dichlorométhane, eau ou des mélanges afférents.

25. Utilisation d'un composé selon une ou plusieurs des revendications 1 à 18 pour la préparation d'organophosphinates.

26. Procédé pour la préparation d'organophosphinates comprenant la réaction d'un composé selon une ou plusieurs des revendications 1 à 18 avec de l'eau ou des solvants contenant de l'eau ou des mélanges de solvants.

27. Procédé selon la revendication 26, **caractérisé en ce que** la réaction est mise en oeuvre entre -40 et 200°C.

28. Utilisation d'un composé selon une ou plusieurs des revendications 1 à 18 en tant que solvant ou additif de solvant, catalyseur à transfert de phase, agent d'extraction, milieu de transfert thermique, substance tensioactive, plastifiant, agent ignifugeant, additif ou sel conducteur et catalyseurs dans des processus chimiques.

29. Electrolytes, cellules électrochimiques, condensateurs ou piles à combustible comprenant au moins un composé selon une ou plusieurs des revendications 1 à 18.
